(19) Europäisches Patentamt
European Patent Office
Office européen des brevets

(11) EP 1 900 825 A1

(12) EUROPEAN PATENT APPLICATION

(43) Date of publication:
**19.03.2008 Bulletin 2008/12**

(51) Int Cl.:
***C12Q 1/68*** *(2006.01)*

(21) Application number: **06120609.0**

(22) Date of filing: **13.09.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **Rheinische Friedrich-Wilhelms-Universität Bonn
53113 Bonn (DE)**

(72) Inventors:
- **Waha, Andreas
53125 Bonn (DE)**
- **Mikeska, Thomas
53639 Königswinter (DE)**
- **Pietsch, Torsten
53359 Rheinbach (DE)**

(74) Representative: **Helbing, Jörg
Patentanwälte
von Kreisler Selting Werner
Postfach 10 22 41
50462 Köln (DE)**

# (54) Sensitive and reproducible method for the determination of MGMT promoter methylation in clinical samples

(57) The present invention provides a method for determination of the methylation degree of the $O^6$-methylguanine DNA methyltransferase gene (*MGMT*) promoter, which is suitable for prediction of the responsiveness of tumors to alkylating agents and for diagnosis or prognosis of cancer. It furthermore provides amplification products and primers which may be used in said method and a kit for performing the method. The method is based on the detection of methylated CpG sites in the *MGMT gene.*



Printed by Jouve, 75001 PARIS (FR)

SEQUENCE LISTING

<110> Rheinische Friedrich-Wilhelms-Universität Bonn

<120> Sensitive and Reproducible Method for the Determination of MGMT Promoter Methylation in Clinical Samples

<130> 062137ep

<160> 10

<170> PatentIn version 3.3

<210> 1
<211> 2340
<212> DNA
<213> homo sapiens

<400> 1
```
cctgctccct ctgaaggctc cagggaagag tgtcctctgc tccctccgaa ggctccaggg      60

aagggtctgt cctcttaggc ttctggtggc ttgcaggtgc agccctccaa tcctcctccc     120

caagcggcct tctgcctata aggacacgag tcatactgga tgaggggccc actaattgat     180

ggcttctgta aagtccccat ctccaaataa ggtcacattg tgaggtactg ggagttagga     240

ctccaacata gcttctctgg tggacacaat tcaactccta ataacgtcca cacaacccca     300

agcagggcct ggcaccctgt gtgctctctg gagagcggct gagtcaggct ctggcagtgt     360

ctaggccatc ggtgactgca gcccctggac ggcatcgccc accacaggcc ctggaggctg     420

cccccacggc cccctgacag ggtctctgct ggtctggggg tccctgacta ggggagcggc     480

accaggaggg gagagactcg cgctccgggc tcagcgtagc cgccccgagc aggaccggga     540

ttctcactaa gcgggcgccg tcctacgacc cccgcgcgct ttcaggacca ctcgggcacg     600

tggcaggtcg cttgcacgcc cgcggactat ccctgtgaca ggaaaaggta cgggccattt     660

ggcaaactaa ggcacagagc ctcaggcgga agctgggaag gcgccgcccg gcttgtaccg     720

gccgaagggc catccgggtc aggcgcacag ggcagcggcg ctgccggagg accagggccg     780

gcgtgccggc gtccagcgag gatgcgcaga ctgcctcagg cccggcgccg ccgcacaggg     840

catgcgccga cccggtcggg cgggaacacc ccgcccctcc cgggctccgc cccagctccg     900

cccccgcgcg ccccggcccc gcccccgcgc gctctcttgc ttttctcagg tcctcggctc     960

cgccccgctc tagaccccgc cccacgccgc catccccgtg cccctcggcc ccgccccccgc   1020

gccccggata tgctgggaca gcccgcgccc ctagaacgct ttgcgtcccg acgcccgcag    1080

gtcctcgcgg tgcgcaccgt ttgcgacttg gtgagtgtct gggtcgcctc gctcccggaa    1140
```

gagtgcggag ctctccctcg ggacggtggc agcctcgagt ggtcctgcag gcgccctcac 1200

ttcgccgtcg ggtgtggggc cgccctgacc cccacccatc ccgggcgagc tccaggtgcg 1260

ccccaagtgc ctcccaggtg ttgcccagcc tttccccggg cctggggttc ctggactagg 1320

ctgcgctgca gtgactgtgg actggcgtgt ggcgggggtc gtggcagccc ctgccttacc 1380

tctaggtgcc agccccaggc ccgggccccg ggttcttcct acccttccat gctgccagct 1440

ttccctccgc cagctgctcc aggaagcttc cagaagcccc tgcgcgggcc ttggcttgca 1500

gcaacccttt agcatactta ggcagagtcc catatttcct tcctgctgga ggccaagttc 1560

taggggcctt ctggttacta tggctggtgt ttgtgtacat cataccctaa ctgtattcat 1620

caacacttag agtaagcaag gctcgctgga gagccacaca cactgggcac cgtaatgtcg 1680

gttataacac cgcagaggag ttctgaacta tgtatttcgc actcctgggt tcatcatctc 1740

ctgaaatctc agggtggtgt ttgctctcag ttgcttcagc tgagtagctg gctttctgtc 1800

ctggaaagca gactttgtac atgtgtgtgc aacctatgcc tgctgagatc atcatcagac 1860

agggaagcgg cttggtccag agagctgttc tcagtagaat gttaagcaca gagagctgag 1920

aattagactg gttatttaca tagacatcca aatagaaacc tatagagtat ctgttaagtc 1980

aggctctccc gtcatctccc ccatccctgg gcaggtgtct aggagatggt tttgttattt 2040

tcagggccct ctcaatggct aaaacactct gggagatgaa caagatttta aaaacccaaa 2100

gcttagcgca cctggtgggt gggggtgtga aaactttgaa ggaaaccgcg tcaagagcct 2160

ggctgattgt taatatcacg ttaactcaga gggccaggat acttgcccag acccggagtc 2220

tgcctgcaag tagcagagga gagctggcct tgctctgccg cgtgtctttc ttcctgggcc 2280

ctctgtctcg ggttggaatt tgaatagtgg agtagtgtct gccacagtca ggggcctgga 2340

<210> 2
<211> 18
<212> DNA
<213> artificial seqeunce

<220>
<223> forward primer for PCR with bisulfite-treated DNA

<400> 2
ggatatgttg ggatagtt 18

<210> 3
<211> 19

<212> DNA
<213> artificial sequence

<220>
<223> reverse primer for PCR with bisulfite-treated DNA

<400> 3
aaactaaaca acacctaaa 19

<210> 4
<211> 36
<212> DNA
<213> artificial sequence

<220>
<223> forward primer for 1st COBRA-PCR

<400> 4
cacgacgttg taaaacgacg atatgttggg atagtt 36

<210> 5
<211> 37
<212> DNA
<213> artificial sequence

<220>
<223> reverse primer for 1st COBRA-PCR

<400> 5
ggataacaat ttcacacagg cccaaacact caccaaa 37

<210> 6
<211> 19
<212> DNA
<213> artificial sequence

<220>
<223> forward primer for 2nd COBRA-PCR

<400> 6
cacgacgttg taaaacgac 19

<210> 7
<211> 20
<212> DNA
<213> artificial sequence

<220>
<223> reverse primer for 2nd COBRA-PCR

<400> 7
ggataacaat ttcacacagg 20

<210> 8
<211> 17
<212> DNA
<213> artificial sequence

<220>
<223> primer for pyrosequencing

<400> 8
cccaaacact caccaaa 17

<210> 9
<211> 20
<212> DNA
<213> artificial sequence

<220>
<223> pyrosequenced sequence

<400> 9
tcrcaaacra tacrcaccrc 20

<210> 10
<211> 14
<212> DNA
<213> artificial sequence

<220>
<223> sirph primer

<400> 10
gtgagtgttt gggt 14

## Description

The present invention provides a method for determination of the methylation degree of the $O^6$-methylguanine DNA methyltransferase gene (*MGMT*) promoter, which is suitable for prediction of the responsiveness of tumors to alkylating agents and for diagnosis or prognosis of cancer. It furthermore provides amplification products and primers which may be used in said method and a kit for performing the method. The method is based on the detection of methylated CpG sites in the *MGMT* gene.

## Background of the Invention

The human *MGMT* ($O^6$-methylguanine DNA methyltransferase, EC 2.1.1.63) gene is located on chromosome band 10q26 and encodes a protein with DNA repair activity. This protein removes alkyl groups from the $O^6$-position of guanine (1, 2) by an irreversible transfer of this group to a cysteine residue in its active site (3, 4). Guanine in the DNA is thereby restored and MGMT sentenced to proteasome-mediated degradation. Due to the stoichiometry of this reaction and the unavoidable fate of the MGMT protein the repair capacity of a cell depends on the amount of MGMT molecules in the nucleus and the cell's capability of re-synthesis. Failure to repair the $O^6$-Alkylguanine ($O^6$-AG) DNA adducts increases mutagenic potential during replication, because $O^6$-AG can be mistaken for adenine and finally mismatched with thymine. This will result in a G:C to A:T transition mutation. In addition, the adducts show a cytotoxic potential by causing DNA double-strand breaks. Both effects frequently induce apoptosis (5).

*MGMT* is a large gene spanning approximately 300 kb, including 5 exons at chromosomal region 10q26 (accession number: NT_008818.15, 2499397-2799297); its mRNA is 866 bp long, encoding 207 amino acids (accession numbers of Ref Seq: NM_002412.2 and NP_002403.1). The DNA sequence and function of the gene product are conserved from prokaryotes to mammals. The gene is expressed in all normal human and mouse cells.

The *MGMT* gene has a TATA-free, GC-rich promoter containing a CpG island, a short stretch of GC-rich sequences associated with promoter regions in approximately half of human genes. The promoter with maximal activity lies 5' of the gene from approximately 1 kb upstream of the transcription initiation site to 200 bp downstream of it (reviewed in: Soejima H et al., Biochem. Cell Biol. 2005, 83:429-437). Usually, this region is designated as "*MGMT* promoter" or "*MGMT* promoter region". It comprises exon 1 of *MGMT,* which plays a crucial role in transcription control.

The MGMT expression level and its activity varies widely depending on tissue and cell type. High expression variability is also observed in tumor tissues *(6).* Brain tumors show low expression, while the activity of MGMT is increased relative to the surrounding normal tissue *(7*, *8*). Expression of MGMT is regulated epigenetically by methylation of the MGMT promoter region. Promoter hypermethylation leads to transcriptional silencing resulting in decreased MGMT protein levels and decreased repair activity. This important regulatory mechanism contributes to loss of MGMT expression in human tumors *in vivo* as first described by Esteller *(9).*

On the other hand, the *MGMT gene* is inducible by DNA damaging treatments such as ionizing radiation, alkylating drugs, or dexamethasone treatment *(10, 11)* when its promoter is unmethylated. It is important to note that these observations were uncovered in rat, mouse, and human cell lines *in vitro* and in rodent tissue *in vivo.* The situation in human tissues is still unknown.

Methylation of the *MGMT* promoter occurs at CpG sites, i.e. at sites wherein C and G are connected by a phosphodiester bond. The cytosine in the CpG site is methylated resulting in 5-methylcytosine. The *MGMT* promoter contains more than 100 CpG sites. 97 of them are clustered in a CpG island (Soejima H et al., Biochem. Cell Biol. 2005, 83:429-437). A CpG island is a region with at least 200 bp which has a GC percentage that is greater than 50% and an observed/expected CpG ratio greater than 0.6 (Gardiner-Garden, M. and Frommer, M., J. Mol. Biol. 1987; 196(2): 261-282). CpG islands are often located around the promoters of frequently expressed genes like housekeeping or repair genes and are usually unmethylated in normal tissues.

Whether a CpG position has been methylated or not can be determined by bisulfite derivatization of the DNA. Cytosine and its methylated counterpart 5-methylcytosine show a different behavior to the treatment with sodium bisulfite on single-stranded DNA. While cytosine residues react with this reagent and are converted to uracil, 5-methylcytosine stays inert under the same conditions. In a subsequent PCR reaction the uracil residues are transcribed to thymine and 5-methylcytosine to cytosine. After cloning and sequencing of the amplicons a comparison with the genomic sequence reveals that a formerly unmethylated CpG-dinucleotide appears as a TpG whereas a methylated one remain as a CpG. Thus, methylated cytosine can be discriminated from unmethylated cytosine.

Determination of CpG methylation in the *MGMT* promoter has proved as a useful tool in the diagnosis and prediction of lung cancer (Palmisano, WA et al., Cancer Res. 2000, 60:5954-5958).

Resistance to chemotherapy is a major complication during treatment of cancer patients with alkylating agents. The epigenetically mediated silencing of the *MGMT* gene in tumors, which results from hypermethylation of its promoter, has been associated with an increased mean survival time in glioma patients that were treated with alkylating agents (*12*, *13*). The high repair activity in tumors with a transcriptionally active *MGMT* gene is believed to protect tumor cells against the cytotoxic effect of these anticancer drugs *(14)*. Thus, predicting the clinical response to chemotherapeutic treatment with alkylating agents is possible based on the methylation status of *MGMT* (*12, 15*, WO 02/27019). Recently it was shown that presence of DNA methylation in the 5'-region of the *MGMT* gene is a predictive marker for favorable outcome in patients with glioblastoma treated with the alkylating agent temozolomide *(15)*. This drug mediates its cytotoxic effect by forming $O^6$-MeG DNA-adducts. MGMT-deficient glioma cells show strong apoptotic response to $O^6$-MeG DNA-adducts upon treatment with temozolomide *(16)*. Therefore, *MGMT* promoter methylation may represent an important epigenetic marker for chemotherapy resistance (*12, 15,* WO 02/27019).

Most of the publications dealing with the detection of *MGMT* methylation use a variant of methylation-specific PCR (MSP) *(17, 18, Palmisano et al., loc. cit.),* which was first adapted for *MGMT* by Esteller (*9,* WO 02/27019). MSP is an important bisulfite-based method for analyzing the DNA methylation status with high sensitivity. This method represents a non-quantitative, cost-efficient analysis. However, MSP bears a significant risk of false positive or false negative results, especially when DNA quality and/or quantity is low, which is often the case in a clinical setting where samples are typically obtained from FFPE (formalin-fixated paraffin-embedded) specimen.

MSP is limited by several constraints. First, mosaic methylation patterns with variable grades of methylation at the primer position can lead to false positive or false negative results. This mis-priming risk further increases when high numbers of PCR cycles or nested primers are used *(18)* as is typically necessary for processing DNA from FFPE samples by MSP. Second, reliable presence or absence of the methylation-specific band depends on several factors that are difficult to guarantee in a clinical setting, such as reproducible amounts of bisulfite-treated DNA used for amplification and use of high-quality genomic DNA for bisulfite treatment. Third, due to multiple factors influencing band strength (such as PCR performance of each MSP reaction and concentration of bisulfite-treated DNA) MSP cannot provide reliable quantitative information on *MGMT* methylation. In conclusion, the use of MSP in a clinical setting is associated with significant uncertainties and accuracy risks inherent in the PCR reaction, especially if applied to a mosaicism methylation target from a heterogeneous cell population. Therefore it should be used with great caution especially if a therapeutic decision is desired. Hence, problems reported by Hegi *et al. (15)* especially on FFPE tumor samples (for only 67% of the samples the MGMT methylation status could be determined by MSP) are not unexpected and further difficulties mentioned in a recent paper *(28)* substantiate the conclusion that MSP is unsuitable for establishment of a robust MGMT marker for clinical settings. Taking the importance of *MGMT* as a predictive marker into account, alternative methods have to be established for reliable analysis of the *MGMT* methylation status.

Alternative techniques for methylation analysis like bisulfite sequencing of several clones, which are more tolerant towards low sample quality than MSP, are semi-quantitative and widely used in basic research setting but are neither cost effective nor fast enough to be implemented for routine clinical diagnosis.

Bisulfite sequencing of single clones is a widely used application to analyze the methylation profile of an amplicon. Its main advantage is the possibility to analyze each single CpG position with high resolution at the level of single alleles. This results in a quantitative methylation profile of the analyzed region when multiple clones are analyzed. However, the experimental procedure of bisulfite sequencing is a time-consuming and expensive task. Furthermore, subsequent processing of the obtained DNA methylation data is tedious and error-prone work which requires an experienced experimenter. The typical number of analyzed sequences is in the range of 10 to 15, which can lead to instable means for mosaic methylation patterns and high standard deviations (as can be seen in Table 2).

Thus, the problem underlying present invention is the provision of an improved method for the determination of *MGMT* promoter methylation which does not possess the drawbacks described above for known methods and which is suitable for application in a clinical setting. The *MGMT* promoter methylation degree may be used as an epigenetic marker for cancer diagnosis and cancer prediction, and for prognosis of the effect of alkylating agents on a cancer.

**Summary of the Invention**

The present invention provides a robust, cost-efficient, and easy-to-use method for the sensitive and quantitative assessment of *MGMT* methylation on isolated DNA, especially genomic DNA. Its general applicability was confirmed on DNA extracted from frozen tissues and formalin-fixed, paraffin-embedded (FFPE) specimens. The method is preferably performed using Pyrosequencing™ or COBRA, more preferably using Pyrosequencing™.

Hypermethylation of the *MGMT gene* alters DNA repair and is associated with longer survival of glioblastoma patients treated with alkylating agents. Methylation of *MGMT* is also found in several other cancer entities. Therefore *MGMT* plays an important role as a predictive epigenetic marker for chemotherapy resistance. To adopt this established correlation into a molecular diagnosis and prognosis procedure, we compared different technologies and different CpG sites with regard to their accuracy in reflecting the *MGMT* methylation.

We used sodium bisulfite treatment to modify genomic DNA from 22 primary glioblastoma multiforme (GBM) tumor samples and 3 white matter biopsies as normal brain controls. The bisulfite derived PCR products were further analyzed by either sequencing of individual clones, or by one of the following methods: COBRA, SIRPH, and Pyrosequencing™ to investigate altered methylation of *MGMT.*

We identified most informative CpG positions in the promoter region of *MGMT* discriminating glioblastoma DNA from normal brain tissues. The results of this comprehensive investigation were used for the development of different assays to assess the methylation state at these positions. Finally, we compared these approaches with regard to their robustness, significance and reproducibility on well-characterized tumor samples.

Due to the reliable (no false positives/negatives), quantitative and sensitive detection of *MGMT* methylation in the method of present invention, its outcome is a reliable basis for decisions on suitable therapy for several tumor entities, especially for the decision whether to use alkylating agents.

Thus, the present invention provides

(1) a method for determination of the methylation degree of the $O^6$-methylguanine DNA methyltransferase gene (*MGMT*) 5' non-coding region ("*MGMT* promoter") in a DNA sample, which comprises

(a) treating the DNA sample with bisulfite;
(b) amplifying a segment ("amplified segment") of the bisulfite-modified DNA obtained in step (a) by PCR using the bisulfite treated DNA as template, wherein the amplified segment comprises at least 20 consecutive bisulfite-modified nucleotides within the region nt 1041 to nt 1260 of the native *MGMT* sequence represented by SEQ ID NO:1 and spans at least three CpG sites ("selected CpG sites"); and
(c) analysing the amplification product and determining the methylation status of the selected CpG sites in the amplified segment;

(2) the method according to embodiment (1) above, wherein the selected CpG sites covered by the amplified fragment comprise either (i) CpG 9 to CpG 12 or (ii) CpG 5, 8 and 9;

(3) the method of embodiment (1) or (2) above, wherein step (c) comprises Pyrosequencing™ or restriction analysis of the amplification product;

(4) a method for prediction of the responsiveness of tumors to alkylating agents which comprises performing the method of any one of embodiments (1) to (3) above, wherein the methylation degree of the selected CpG sites is indicative for the responsiveness of the tumor;

(5) a method for diagnosis or prognosis of a cancer which comprises performing the method of any one of embodiments (1) to (3) above, wherein the methylation degree of the selected CpG sites is indicative for a cancer or cancer risk;

(6) an isolated DNA molecule which is

(i) the amplification product as obtainable by step (b) according to any one of embodiments (1) to (3) above;
(ii) a primer for the PCR reaction in step (b); or
(iii) a primer for the Pyrosequencing reaction of embodiment (3) above; and

(7) a kit for performing the method of any one of embodiments (1) to (5) above.

## Short Description of the Figures

Fig. 1. Strategy for the optimization of *MGMT* promoter methylation detection as an epigenetic marker for chemotherapy resistance.
Fig. 2. (a) General map of the CpG island (CGI) spanning the *MGMT* promoter region including exon 1 of the *MGMT gene.* (b) Nested PCR approach used by Hegi *et al. (15).* The second step (2°) used the methylation sensitive primer established by Esteller *et al. (9).* (c) PCR product used for bisulfite sequencing of single clones, SIRPH, and Pyrosequencing™ (biotin label not shown). (d) Double labeled PCR product used for the COBRA assay. Circles: CpG positions.
Fig.3. Hierarchical clustering of samples by their *MGMT* promoter methylation profiles (methylation averages and standard deviations over all individual clones at CpG positions 1 to 25). Dendrogram using Average Linkage between Groups.
Fig. 4. Methylation profile (methylation averages over all individual clones) of the low methylation tumor subclass.
Fig. 5. Methylation profile (methylation averages over all individual clones) of the high methylation tumor subclass.

Fig. 6. Methylation pattern obtained by bisulfite sequencing of single clones. a: sample 08, b: sample 14, c: sample 16, d: sample 18, e: sample 21, f: sample 22, g: sample 24, h: sample 13, i: sample 20, and j: control sample 3. Filled circles correspond to methylated CpG positions, unfilled circles correspond to unmethylated CpG positions, and the vertical lines without a circle correspond to CpG positions not determined in sequence. The diagrams were generated with the the BiQ Analyzer software (25).

Fig. 7. Typical chromatograms obtained in the SIRPH assay for an unmethylated sample, sample 15 (a), and for a methylated sample, sample 21 (b). UP: signal of the un-extended primer; +C and +T: signal of the ddCTP and ddTTP extended primer, respectively. The signal seen in the beginning of the chromatogram is due to loading the sample on the column.

Fig. 8. Typical pyrograms obtained for an unmethylated sample, sample 25 (a), and for a methylated sample, sample 14 (b). Each box represents one of the four CpG positions interrogated by the Pyrosequencing™ assay, starting on the left side with CpG 12, due to reverse sequencing the upper strand of the PCR product. As a consequence a C/TpG position appears as a CpG/A. The incorporation of the base guanine represents in this context the methylated fraction (arrows) and the base adenine the unmethylated fraction, respectively.

Fig. 9. Titration curves obtained for each individual CpG position interrogated by the Pyrosequencing™ assay. The standard deviation of the data point is shown by a vertical bar. The straight line reflects the theoretical curve. Exp.: expected, obs.: observed.

Fig. 10. Marker scores for all three methods plotted against overall methylation level. (A) The CO7 marker based on the analysis of CpGs 1, 2, 5, 8, and 9. (B) Marker SI01 based on the analysis of CpG13, while (C) the Py15 marker based on the analysis of the CpG positions 9, 10, 11, and 12.

**Sequence Listing - Free Text**

| SEQ ID NO: | designation |
|---|---|
| 1 | part of human MGMT comprising the MGMT promoter |
| 2 | MGMT-Bis fw primer |
| 3 | MGMT-Bis rev primer |
| 4 | MGMT CO-1 fw primer |
| 5 | MGMT CO-1 rev primer |
| 6 | MGMT CO-2 fw primer |
| 7 | MGMT CO-2 rev primer |
| 8 | Pyrosequencing primer |
| 9 | pyrosequenced segment of SEQ ID NO:1 |
| 10 | SIRPH-SNuPE-primer |

**Detailed Description of the Invention**

Present invention provides an improved analysis method of MGMT promoter methylation for application in a clinical setting as an epigenetic marker.

SEQ ID NO:1 represents a segment of the native, unmethylated MGMT gene and its 5'region. It corresponds to nt 45866 to 48205 of GenBank accession number AL355531. The CpG positions which are relevant for present invention are located in SEQ ID NO:1 as follows:

CpG 01 : bp 1044+1045
CpG 02 : bp 1046+1047
CpG 03 : bp 1057+1058
CpG 04 : bp 1064+1065
CpG 05 : bp 1069+1070
CpG 06 : bp 1072+1073
CpG 07 : bp 1076+1077

CpG 08 : bp 1086+1087
CpG 09 : bp 1088+1089
CpG 10 : bp 1093+1094
CpG 11 : bp 1098+1099
CpG 12 : bp 1104+1105
CpG 13 : bp 1125+1126
CpG 14 : bp 1130+1131
CpG 15 : bp 1136+1137
CpG 16 : bp 1146+1147
CpG 17 : bp 1159+1160
CpG 18 : bp 1164+1165
CpG 19 : bp 1176+1177
CpG 20 : bp 1192+1193
CpG 21 : bp 1203+1204
CpG 22 : bp 1206+1207
CpG 23 : bp 1209+1210
CpG 24 : bp 1221+1222
CpG 25 : bp 1242+1243
CpG 26 : bp 1246+1247
CpG 27: bp 1259+1260

The *MGMT* promoter CpG island to which the following part refers is represented by nt 401 to 1200 of SEQ ID NO:1.

Exon 1 of *MGMT* is represented by nt 1041 to 1110 of SEQ ID NO:1.

The term "5'non-coding region" has several synonyms, namely "*MGMT* promoter" and "*MGMT* promoter region", which are used throughout the present invention. These terms do all designate the 5'region of the *MGMT* gene which does control *MGMT* transcription and whose effect on transcription can be influenced by CpG methylation. In the context of present invention, the MGMT promoter consists of the sequence represented by SEQ ID NO:1.

In the following, bp and nt will be used synonymously for indication of positions in a nucleic acid sequence. Therefore, a position indication for double stranded DNA by nt always includes the basepair and the complementary nucleotide on the complementary strand.

The amplification reaction in step (b) of embodiment (1) is performed using the bisulfite-modified DNA obtained by the bisulfite treatment of the initial DNA sample in step (a). The outcome of the bisufite treatment depends on whether a C was methylated or not in the initial DNA sample. Thus, the nucleotide sequence of the bisulfite-treated DNA will differ from the bisulfite-untreated DNA sequence: every unmethylated C has reacted with bisulfite and was converted to uracil, every 5-methylcytosine stayed inert under the same conditions. In a subsequent PCR reaction the uracil residues are transcribed to thymine and 5-methylcytosine to cytosine. As the number of methylated Cs in the samples will differ from sample to sample (generally, the Cs in DNA samples from healthy persons are generally unmethylated, whilst a considerable portion of the Cs in DNA samples from tumors is methylated), there are more than one possible nucleotide sequences resulting from the bisulfite treatment of the *MGMT* gene. Thus, in order to simplfy comparison, the nucleotide positions in DNA sequences resulting from the bisulfite treatment ("bisulfite treated counterpart" of the initial DNA) and from PCR amplification reactions perforemd on said bisulfite-treated counterpart will be given as the corresponding positions in the native *MGMT* sequence represented by SEQ ID NO:1. The tern "bisulfite-treated counterpart" within the ambit of the invention also includes the sequence complementary to said counterpart, i.e. the amplification product of both bisulfite treated strands. It should be kept in mind that in the bisulfite-treated DNA the C may be replaced by a U and that in the amplification products the CG basepair at the site of such U is replaced by TA.

"Selected CpG sites" in the context of present invention contribute to the determination of methylation status of the *MGMT* gene because they are analyzed in step (c) in order to establish whether they were methylated or not.

In a preferred aspect of embodiment (1), the at least three selected CpG sites covered by the amplified segment are selected from the group consisting of the CpG sites at positions CpG 1, CpG 2, CpG 3, CpG 4, CpG 5, CpG 6, CpG 7, CpG 8, CpG 9, CpG 10, CpG 11, CpG 12 and CpG 13. 3. More preferably, they are selected from the group consisting of CpG 3 to 13, even more preferably of CpG 5 to 12. Most preferably, they are (i) CpG 9 to 11, (ii) CpG 9 to 12, (iii) CpG 5, 8 and 9 or (iv) CpG 1, 2, 5, 8 and 9. The amplified segment may span additional CpG sites.

In a further preferred aspect of embodiment (1), the amplified segment in step (b) spans at least 4 selected CpG sites.

Moreover, ist is preferred that the amplified segment comprises at least 20 consecutive nucleotides of the bisulfite-treated counterpart of the sequence represented by nt 1041 to 1105 of SEQ ID NO:1. More preferably, is comprises at least the bisulfite treated counterpart of the sequence represented by nt 1041 to 1089 or nr 1086 to 1105 of SEQ ID NO:1.

The amplification step (b) of embodiment (1) is performed by PCR. The primer pair ("first primer pair") used in said PCR ("first PCR") does preferably not amplify bisulfite-untreated genomic DNA. In other words, the primers or at least one of the primers are complementary to a segment of SEQ IN NO:1 after the bisulfite treatment of said segment ("bisulfite treated counterpart"), but not beforehand. Such primers are selective for the bisufite-treated region of interest of SEQ ID NO:1.

The first primer pair consists of a forward and reverse primer. In a preferred aspect of embodiment (1), the forward primer is complementary to at least 10 consecutive nucleotides of nt 806 to 822, 931 to 954, 1026 to 1043, or 1105 to 1124 of SEQ ID NO:1 or its bisulfite-treated counterpart, and the reverse primer is complementary to at least 10 consecutive nucleotides of nt 1105 to 1124, nt 1250 to 1296, 1298 to 1323, 1325 to 1345, 1361 to 1401, 1410 to 1447 or 1449 to 1482 of SEQ ID NO:1 or its bisulfite-treated counterpart. Preferably, it is complementary to the bisulfite counterpart of the segments listed in the previous sentence, more preferably it binds exclusively to said counterpart.

In any of embodiments (1) to (3), the method may comprise an additional step after step (c), namely a statistical analysis of the methylation scores for the selected CpGs (see also Example 2.7). This allows the separation of DNA samples containing unmethylated *MGMT* from DNA samples containing methylated *MGMT*. It furthermore provides a confidence estimate, i.e. shows the reliability of the methylation prediction. Analysis of selected CpG sites 9, 10, 11 and 12 is the most reliable means for determination of *MGMT* methylation, followed by analysis of CpG 5, 8 and 9 (Fig. 10). The accessibility of the CpG positions 9 to 12 by Pyrosequencing™, which reflects more accurately the overall methylation status than the other CpGs studied by COBRA and SIRPH, puts the Pyrosequencing™ approach in a clear advantage over the other two approaches. COBRA follows in second place.

Since the CpG positions 9 to 12 are four CpG positions the analysis of all of them is more tolerant towards noise than the analysis of less CpG positions. Simulation showed that flipping any one position to zero or to one (which can theoretically happen due to rare C-T-SNPs at the analyzed CpG dinucleotide) can only convert a clearly methylated / unmethylated sample into a doubtful prediction or vice versa, but cannot - for none of the samples we analyzed - convert a clear methylated case into a clear unmethylated case or vice versa. Therefore, the analysis of CpG positions 9 to 12 and, to a lesser extent, the analysis of CpG positions 5, 8 and 9 as described in the Examples section provide the necessary statistical robustness to cope with doubtful and intermediate cases.

In one especially preferred aspect, the statistical analysis is performed using the following classification formulae:

CpGs 1, 2, 5, 8 and 9:

$$Score = -339.385 \cdot CpG_{1and2} + 196.192 \cdot CpG_{8and9} + 137.296 \cdot CpG_{5} - 21.803\ ;$$

CpGs 9 to 12:

$$Score = 21.330 \cdot CpG_{9} + 24.806 \cdot CpG_{10} + 18.637 \cdot CpG_{11} + 21.503 \cdot CpG_{12} - 20.197\ ,$$

where CpG variables refer to the measured methylation score at each position. Overall positive scores predict promoter methylation, negative scores the absence of promoter methylation. A an unexpected negative coefficient at CpG position 1/2 was observed, a high methylation level at this position is not a good predictor of a high overall methylation level.

For all formulae, the resulting score for any single tumor not only gives a prediction of the overall promoter methylation state but also provides a confidence estimate: the further the score deviates from zero, the safer is the prediction.

For CpGs 1, 2, 5, 8 and 9 scores below -15 are highly indicative of overall absence of methylation, while scores above 40 are consistently associated with overall promoter methylation. Between these safety margins, no clear conclusion is possible. For CpGs 9 to 12, safe prediction of unmethylated promoter state is possible below -10, while methylation is present at scores above 10.

It is to be noted that the known method for determination of *MGMT* methylation, MSP, is not focussed on CpG 9 to 12, but on other regions of the *MGMT* gene. Especially CpG 1, 2, 5, 8, 9, 10, 11 and 12 differ from the positions known as indicative for *MGMT* promoter methylation in MSP. Furthermore, MSP does not allow a quantitative determination of the methylation degree.

The determination of methylation of CpG 9 to 11, preferably 9 to 12 is of utmost preference in present invention for two further reasons: In cases wherein all of them are methylated, this allows the conclusion that the CpGs in the CpG island of *MGMT* are methylated in a degree which prohibits the transcription of *MGMT*. And said CpG postitions are predominantly methylated in tumor tissue, but not in healthy tissue.

One especially preferred aspect of present invention is the method of embodiment (3), wherein the analysis step (c) is performed using Pyrosequencing. This analysis method allows the simultaneous analysis of several CpG positions in a range of up to 30 bp and which generates quantitative results for each individually analyzed CpG position. Rapid and parallel processing of a large number of samples is a very important advantage of this technology.

The Pyrosequencing method is limited due to the need of an appropriate primer for extension. The Pyrosequencing primer is preferably designed to minimize the amount of not properly bisufite-converted alleles. This is achieved by incorporating a bisulfite-conversion specific thymine residue on the 3'-end, which helps to avoid mis-priming of residual unconverted DNA which may lead to a false positive interpretation.

In the method using Pyrosequencing, step (b) comprises only one PCR amplification reaction. This minimizes the time, equipment and effort necessary for performing the method, especially in comparison to MSP.

The method using pyrosequencing is preferably performed with an amplified segment comprising at least 20 consecutive nt of the bisulfite-treated counterpart of SEQ ID NO:1. More preferably, the segment has a maximum length of 200 nt, even more preferably of up to 100 nt. However, a segment with 20 nt length is sufficient.

Furthermore, the amplified segment preferably comprises or consists of the sequence represented by nt 1086 to 1105 of the bisulfite-treated counterpart of SEQ ID NO:1.

Finally, in the method using Pyrosequencing, the selected CpG sites comprise at least three CpG sites selected from CpG 9 to 12, and more preferably comprise all 4 CpG sites CpG 9 to 12. The analysis of the methylation status of CpG 9 to 12 (and no further CpGs) is sufficient for a reliable statement on the methylation of the *MGMT* promoter. It is even sufficient for a reliably determination of the methylation status of the *MGMT* when CpG 9 to 12 are the only CpGs whose methylation status is determined. However, if three of the four sites are methylated or if three of the sites are not methylated, this also leads to a reliable statement. Thus, the analysis of three CpG sites selected from CpG 9 to 12, especially of CpG 9 to 11, is also preferred.

There are several preferred aspects of the method using Pyrosequencing:

In one preferred aspect, in the primer pair used in the amplification step (b) the forward primer is complementary to at least 10 consecutive nucleotides of nt 1026 to 1043 of SEQ ID NO:1 or its bisulfite-treated counterpart, and the reverse primer is complementary to at least 10 consecutive nucleotides of nt 1250 to 1296, preferably of nt 1260 to 1296 of SEQ ID NO:1 or its bisulfite-treated counterpart. Preferably, it is complementary to the bisulfite counterpart of the segments listed in the previous sentence, more preferably it binds exclusively to said counterpart. More preferably, the primers are selected from the group consisting of the sequences represented by SEQ ID NOs:2 and 3, sequences comprising SEQ ID NOs: 2 or 3 and also sequences comprising or consisting of SEQ ID NOs:2 or 3 which are truncated by up to three nucleotides at their 5' and/or 3'-end.

In a second preferred aspect, in step (c) of the method a segment comprising at least consecutive nucleotides nt 1088 to 1098 of the bisulfite-counterpart of SEQ ID NO:1, or of the reverse strand of said segment is pyrosequenced. Preferably, the segment comprises nt 1088 to 1105, more preferably nt 1087 to 1106 or nt 1086 to 1105 of the bisulfite-treated counterpart of SEQ ID NO: 1.

The pyrosequencing primer is complementary to at least 10 nt downstream of the pyrosequenced segment. More preferably, it comprises at least 10 consecutive nucleotides of the (+) or the (-) strand of the amplification product resulting from the bisulfite treated counterpart of nt 1105 to 1124, preferably nt 1107 to 1123 of SEQ ID NO: 1. Preferably, it binds exclusively to said counterpart. Most preferred, it is selected from the group consisting of the sequence represented by SEQ ID NO:8, sequences comprising SEQ ID NO:8 and also sequences comprising or consisting of SEQ ID NO:8 which are truncated by up to three nucleotides at their 5' and/or 3'-end.

The second especially preferred aspect of present invention is the method of embodiment (3), wherein the analysis step (c) comprises a restriction digestion of the amplification product and subsequent analysis of the restriction products. A synonym for this method in the context of present invention is COBRA ((***Co***mbined ***B***isulfite ***R***estriction ***a***nalysis). However, of the two methods of embodiment (3), the method comprising the Pyrosequencing analysis is preferred.

In COBRA, the selected CpG sites preferably comprise at least CpG 5, 8 and 9, more preferably comprise CpG 1, 2, 5, 8 and 9. The analysis of the methylation status of CpG 5, 8 and 9 (and no further CpGs) is sufficient for a reliable statement on the methylation of the *MGMT* promoter. However, as the use of certain restriction endonucleases (like the ones used in the examples section) which cut at CpG 8/9 inevitably leads to cuts at CpG 1/2, the selected CpG sites preferably comprise CpG 1, 2, 5, 8 and 9.

Furthermore, COBRA preferably comprises in step (b) two PCR reactions: The first PCR reaction is performed with a first primer pair using the bisulfite-treated template obtained from step (a). The second PCR reaction is performed with a second primer pair using the amplification product of the first PCR reaction and a second primer pair. The first primer pair contains 5'-tails of at least 10 nt length which are complementary to each other and identical to the two second primer sequences. In the second primer pair, one primer is labelled at its 5' tail with one dye molecule (first label) and the second primer is labelled at its 5' tail with a different dye molecule (second label). The first and/or second label allow direct optical detection, such as by detection of fluorescence, chemiluminescence or the like, provided that said second detectable label differs from said first detectable label in order to allow separate detection.

In the primer pair used in the first PCR of COBRA, the forward primer is preferably complementary to at least 10 consecutive nucleotides of nt 1026 to 1043 of SEQ ID NO:1 or its bisufite-treated counterpart, and the reverse primer is preferably complementary to at least 10 consecutive nucleotides of nt 1105 to 1124, nt 1250 to 1296, 1298 to 1323, 1325 to 1345, 1361 to 1401, 1410 to 1447 or 1449 to 1482 of SEQ ID NO:1 or its bisulfite treated counterpart, more preferably of nt 1105 to 1124 of SEQ ID NO:1 or its bisulfite treated counterpart. Preferably, it is complementary to the bisulfite counterpart of the segments listed in the previous sentence, more preferably it binds exclusively to said counterpart. Most preferred primers of the first primer pair are selected from the group consisting of the sequences represented by SEQ ID NOs:6 and 7, sequences comprising SEQ ID NOs:6 or 7 and also sequences comprising or consisting of SEQ ID NOs:6 or 7 which are truncated by up to three nucleotides at their 5' and/or 3 '-end.

The primers of the primer pair used in the second PCR ("second primer pair") are preferably without binding site in the initial DNA sample. More preferably they are also without binding site in the bisulfite-treated counterpart of the initial DNA sample or the amplification product thereof. Thus, they do only bind to each other in the second PCR. Suitable second primer pairs are the non-matching M13 tail primer pair (SEQ ID NOs:6 and 7; derived from bacteriophage M13) or primer pairs comprising at least 10 consecutive nucleotides thereof. Moreover, the primers of the second primer pair are preferably at least 13 nt long.

It is also preferred that the labels at the 5 'ends of the second primer pair are state fluorescent or luminescent dye molecules for DNA as known in the art, preferably fluorescein derivatives, more preferably FAM (6-Carboxyfluorescein) and/or JOE (2,7-dimethoxy-4,5-dichloro-6-carboxyfluorescein).

The COBRA method is preferably performed with an amplified segment comprising at least 48 consecutive nt of the bisulfite-treated counterpart of SEQ ID NO: 1. More preferably, the segment has a maximum length of 200 nt, even more preferably of up to 100 nt. However, a segment with 48 nt length is sufficient.

Furthermore, the amplified segment preferably comprises or consists of the sequence represented by nt 1041 to 1089 of the bisulfite-treated counterpart of SEQ ID NO:1.

Step (c) in COBRA is preferably performed on the amplification product of the second PCR reaction described above. The amplification product which is analyzed in step (c) is preferably labelled with two dye molecules which are different from each other, wherein one dye molecule is covalently linked to the 3' end and the other to the 5' end of the amplification product. More preferably, said amplification product is the result of the second PCR in step (b) using the labelled primers as defined above for the second primer pair.

Finally, in a further preferred aspect of COBRA, at least one restriction endonuclease used in the restriction digestion of step (c) has a recognition site which spans at least one of the selected CpG sites and does recognize only a recognition site containing CpG at the CpG site in the amplification product obtained by step (b). The recognition sites of the endonucleases are preferably comprising CpG 1, 2, 5, 8 and/or 9 or their counterparts resulting fro the bisulfite treatment and the subsequent PCR amplification. More preferably, they comprise one of said CpG and one, two, or three flanking nucleotides on one or on each side of the CpG. Thus, they most preferably are located in nt 1041 to 1048, nt 1043 to 1050, nt 1066 to 1073, nt 1083 to 1090 and/or nt 1085 to 1092 of SEQ ID NO:1. Utmost preferred are restriction endonucleases having recognition sites comprising or consisting of TCGA or CGCG, including Taq$^{\alpha}$I and BstUI (which are the most preferred), Bst1236I (FmuDII), AccII, BstFNI, MvnI (the latter four cut like BstUI) and TaqI (cuts like Taq$^{\alpha}$I).

The DNA sample used in embodiments (1) to (3) is preferably genomic DNA. The method according to the invention is so robust that the use of this rather low quality DNA which comprises mostly non-*MGMT* sequences is tolerated and does not falsify the result.

The DNA sample is preferably isolated from mammalian, more preferably from human tissue. Any mammalian DNA can be used in the method according to the invention, but of course human DNA is of the utmost clinical interest. Furthermore, said tissue may be freshly prepared (e.g. is a biopsy sample) or may be a sample which has been stored before application of the method of embodiment (1). Notably, the method allows the determination of the *MGMT* methylation grade in kryopreserved or formalin-fixed tissue. This is advantageous, as it allows statements on samples which were not kryopreserved, were stored in pathological collections and archives or were taken in the far past.

The DNA sample is in a further preferred aspect of embodiment (1) isolated from a tumor tissue. Preferably, said tumor tissue is tissue isolated from glioma (including glioblastoma multiforme, low grade astrocytoma gliobastoma, oligodendroglioma), diffuse large B-cell lymphoma (DLBLC), colorectal carcinoma, non-small cell lung carcinoma, lymphoma, head and neck carcinoma and brain tumors (including anaplastic astrocytoma, medulloblastoma, neuroblastoma), more preferably tumor tissue isolated from glioma, colorectal carcinoma and DLBLC, most preferably tumor tissue isolated from malignant glioma, especially glioblastoma, and DLBLC.

In a preferred aspect of embodiment (4), a methylation degree of 75 % or more, preferably of 90 % of the selected CpG sites indicates that the tumor is susceptible to alkylating agents.

Embodiment (5) allows prognosis and diagnosis of cancer by the method of any of embodiments (1) to (3).

However, any DNA analytic method allowing the discrimination between methylated and unmethylated CpG 9 to 11, preferably CpG 9 to 12, e.g. hybridisation with appropriate probes, is suitable as the mandatory first step towards a reliable diagnosis/prognosis.

The primer of embodiment (6)(ii) is any primer of the first primer pair or second primer pair as described above. In a preferred aspect of embodiment (6)(ii), the primer is complementary to at least 10 consecutive nucleotides of nt 806 to 822, 931 to 954, 1026 to 1043, 1105 to 1124, nt 1250 to 1296, 1298 to 1323, 1325 to 1345, 1361 to 1401, 1410 to 1447 or 1449 to 1482 of SEQ ID NO:1 or the bisulfite-treated counterpart of SEQ ID NO:1. More preferably, the primer is complementary to at least 10 consecutive nucleotides of nt 1026 to 1043, nt 1105 to 1124, nt 1250 to 1296, 1298 to 1323, 1325 to 1345, 1361 to 1401, 1410 to 1447 or 1449 to 1482 of SEQ ID NO:1 or the bisulfite-treated counterpart of SEQ ID NO:1. Even more preferred, it is complementary to the bisulfite counterpart of the segments listed in the previous two sentences, more preferably it binds exclusively to said counterpart. Most preferably, the primer is complementary to at least 10 consecutive nucleotides of nt 1026 to 1043, 1105 to 1124 or nt 1260 to 1296 of the bisulfite-treated counterpart of SEQ ID NO: 1.

The primer may comprise a tail as described above and exemplified for M13, and it may be labeled with a label as outlined above.

The most preferred primers of embodiment (6)(ii) are selected from the group consisting of the sequences represented by SEQ ID NOs:2 and 3, sequences comprising SEQ ID NOs:2 or 3 and also sequences comprising or consisting of SEQ ID NOs:2 or 3 which are truncated by up to three nucleotides at their 5' and/or 3'-end. More preferably, they are selected from the group consisting of the sequences represented by SEQ ID NO:3, sequences comprising SEQ ID NO: 3 and also sequences comprising or consisting of SEQ ID NO: 3 which are truncated by up to three nucleotides at their 5' and/or 3'-end.

The Pyrosequencing primer of embodiment (6)(iii) is preferably a primer as outlined above as suitable for the preferred pyrosequenced segment, more preferably a primer selected from the group consisting of the sequence represented by SEQ ID NO:8, sequences comprising SEQ ID NO:8 and also sequences comprising or consisting of SEQ ID NO:8 which are truncated by up to three nucleotides at their 5' and/or 3'-end.

The kit of embodiment (7) preferably comprises one or more of the primers of embodiment (6) and/or one or more of the restriction endonucleases indicated as suitable for COBRA above.

Several of the preferred aspects of present invention are described in more detail in the following sections:

Resistance to chemotherapy is a major complication during treatment of cancer patients. Hypermethylation of the *MGMT*-gene alters DNA repair and is associated with longer survival of glioblastoma patients treated with alkylating agents. Methylation of *MGMT* is also found in several other cancer entities. Therefore MGMT plays an important role as a predictive epigenetic marker for cancer and for chemotherapy resistance. To adopt this established correlation into a molecular diagnostic procedure, we compared different technologies with regard to their sensitive and reproducible detection of methylation in frozen and paraffin embedded tissues. DNA was extracted either from frozen or formalin-fixed, paraffin-embedded (FFPE) gliobastoma samples. Following bisulfite treatment and PCR, sequencing of individual clones, COBRA, SIRPH, and Pyrosequencing was used to investigate altered methylation of *MGMT*. We identified most informative CpG positions in the promoter region discriminating glioblastoma DNA from normal brain tissues. The results of this comprehensive investigation were used for development of different assays to assess the methylation state at these positions. Finally, we compared these approaches with regard to their robustness, significance and reproducibility. Based on the results, we have established a robust, cost-efficient, and easy-to-use clinical diagnosis platform for the sensitive and quantitative assessment of *MGMT* methylation as a reliable clinical tool.

Positions in the *MGMT* promoter were verified that are reliably correlated with the overall methylation state of the promoter (which has been shown to be highly associated with chemotherapy resistance) and - at the same time - are accessible to one of the three techniques that are applicable in a clinical setting: COBRA (***Co***mbined ***B***isulfite ***R***estriction ***A***nalysis) (19), SIRPH (***S***NuPE (single nucleotide primer extension) ***I***P-***RP H***PLC (ion pair RP HPLC)) *(20),* and Pyrosequencing™ *(21).* Second, we systematically optimized each method for robust determination of MGMT promoter methylation and tested its performance on well-characterized tumor samples. Finally, the results for said three techniques were compared with respect to reliability, expenditure, and applicability for molecular diagnostics.

We first established detailed *MGMT* promoter methylation data for 22 tumor samples by bisulfite sequencing data (Fig. 6) and observed two distinct subclasses plus two intermediate cases. The first subclass exhibited entirely unmethylated alleles with sporadic cases of methylated CpG positions (e.g. Sample 13, Fig. 6h, and normal brain 3, Fig. 6j). In contrast, tumors in the second subclass demonstrated high and variable methylation levels (e.g. Sample 22, Fig. 6f). Alleles were on average densely methylated but with variation between CpG positions and between different alleles. In addition, we observed two cases with heterogeneous patterns, characterized by few alleles showing relatively strong methylation in a large number of weak methylated or unmethylated alleles (Samples 8 and 16, Fig. 6a, 6c). Using these well-characterized samples, we benchmarked the predictiveness of all candidate markers.

Restriction-based methods such as COBRA are typically used when the methylation profile is known and the most informative positions or regions in an amplicon have been identified. With the help of restriction endonucleases that recognize DNA methylation at specific sites, the informative positions can be analyzed. The recognition of the restriction site and the number of cutting sites are the most limiting factors of this technology because in many cases there is no enzyme available that fulfills the demands for analyzing the position(s) of interest. Furthermore the reaction conditions for a suitable enzyme must be carefully established, to guarantee a complete digestion without any unwanted side effects. On the other hand, when an appropriate enzyme or enzyme-combination is suitable it results in a cost-effective assay to obtain quantitative or semi-quantitative information of the methylation level for the screened CpG position. Another advantage of COBRA is the possibility to process a large series of samples in parallel.

The established COBRA assay is suitable for both high-quality and low-quality DNA material, for two reasons: First the amplified region spans the first 12 CpG sites with a total length of only 97 bp. Second we used a pseudo-nested PCR approach to enrich the yield of amplicons without saturating the PCR reaction. The chosen system with the restriction endonucleases *Taq*$^{\alpha}$*I* and *BstUI* avoids misinterpretation of the obtained results. The use of two enzymes minimizes errors if the condition for one enzyme is less optimal. Furthermore *Taq*$^{\alpha}$*I* is in its given sequence context a bisulfite conversion specific enzyme. That is, its recognition site TCGA is only present when the first base of the cleavage site, in the genomic DNA a cytosine residue, is converted by the bisulfite treatment and appears finally as thymine. Therefore it eliminates not properly converted alleles and minimizes the rate of false positive methylation.

To assess the predictiveness of the optimized COBRA marker (CO7), we plotted its score for 14 tumor samples against the overall *MGMT* promoter methylation (Figure 10). The results show high separation between the two subclasses with high and low methylation, indicating that the CO7 marker is suitable for robust detection of *MGMT* promoter methylation. Tumor sample 16, which we classified as a methylated borderline case, reflects this behavior also in this diagram, falling between the unmethylated and the methylated samples.

The SIRPH method is limited to the analysis of a subset of CpG positions since a primer of at least 6 bases with no overlapping CpGs adjacent to the relevant position is required for extension. Furthermore the use of more than one primer require considerable efforts of optimization to obtain best resolution of the informative signals in the chromatogram of a dHPLC run. Further disadvantages are the requirement of an expensive dHPLC machine that requires daily maintenance and the serial processing of the samples.

The scores for the SIRPH SI01 marker, which is based on CpG 13, showed no clear separation of methylated and unmethylated cases (Fig. 10). Although the unmethylated samples were grouped together to some degree, the methylated samples were distributed over a broad range, thereby undermining reliable classification. The problems of the SI01 marker were particularly obvious for the unmethylated sample number 20 and the methylated sample number 18, which shared the same value. This actually reflects the limitation of using some restricted CpG sites as position 13 that do not reflect accurately the total methylation of the whole promoter region. Moreover this limits the SIRPH approach to those regions where suitable SNuPE primers are accessible.

Pyrosequencing™ is a powerful method which is capable of analyzing several CpG positions simultaneously (up to 30 bp amplicon length) and which generates quantitative results for each individually analyzed CpG position. Rapid and parallel processing of a large number of samples is a very important advantage of this technology. The method is limited due to the need of an appropriate primer for extension. The Pyrosequencing™ primer was designed to minimize the amount of not properly converted alleles. This was achieved by incorporating a conversion specific thymine residue on the 3'-end, which helps to avoid mis-priming of residual unconverted DNA which may lead to a false positive interpretation.

Plotting the score of the optimized Pyrosequencing™ marker (Py15) against overall *MGMT* promoter methylation (Fig. 10) shows that this marker provides excellent separation between methylated and unmethylated cases with high confidence scores for both groups. As for the COBRA marker sample 16 again exhibited its character of a borderline case. Overall, Py15 is the most accurate and most robust of all *MGMT* methylation markers that were explored in this study and is therefore most suitable for use in clinical settings. However, since Pyrosequencers are not yet widely available, we can also recommend the COBRA-based CO7 marker as the second-best method to assess *MGMT* promoter methylation.

The main focus of our work was to translate the important epigenetic marker *MGMT* into a robust and easy-to-use clinical diagnosis tool that can be applied to DNA extracted from clinical samples. The marker's robustness was confirmed on FFPE specimen, opening up the possibility to accurately investigate *MGMT* methylation in archival tissues. The Pyrosequencing™ assay of present invention is suitable for a clinical application and allows accurate and sensitive identification of *MGMT* methylation for subsequent therapeutic decisions.

The present invention is described in more detail by reference to the following examples. It should be understood that these examples are for illustrative purpose only and are not to be construed as limiting the invention.

## Examples

In the following examples, standard techniques of recombinant DNA technology and molecular biology were used that were described in various publications, e.g. Sambrook et al. (2001), Molecular Cloning: A Laboratory Manual, 3rd edition, Cold Spring Harbor Laboratory Press, or Ausubel et al. (1987), Current Protocols in Molecular Biology, Current Protocols in Immunology, Current Protocols in Protein Science, and Current Protocols in Cell Biology, edition as of 2002, Wiley Interscience. Unless otherwise indicated, all enzymes, reagents, devices and kits were used according to the manufacturers' instructions.

Example 1: Materials and Methods

1.1 DNA samples

Frozen tissue samples were collected from 22 patients with primary gliobastoma multiforme (GBM) (WHO IV) treated at the Deparments of Neurosurgery at the Medical Centers in Bonn (Germany) and Düsseldorf (Germany). The histological typing of the tissues was done according to the World Health organization (WHO) grading system of brain tumors using standard histological and immunohistological methods *(22).* Tissues were selected for extraction of DNA after careful examination on haematoxylin and eosin staining of corresponding frozen sections to exclude contaminating necrotic debris or normal brain tissue. Molecular genetic analyses were performed on samples showing an estimated tumor cell content of at least 80%. Genomic DNA was extracted from tumor tissues using standard proteinase K digestion and phenol/chloroform extraction *(23).* Three white matter biopsies served as normal brain controls. All patients gave written informed consent for these studies.

1.2 Bisulfite treatment

Genomic DNA (300 ng) was subjected to bisulfite conversion with the EpiTect® Bisulfite Kit (Qiagen) according to the manufacturers' instructions.

1.3 Bisulfite sequencing

The primers used for amplification of bisulfite-treated DNA were MGMT-Bis fw-5'-GGA TAT GTT GGG ATA GTT-3' (*24*) (SEQ ID NO:2) and MGMT-Bis rev-5'-AAA CTA AAC AAC ACC TAA A-3' (SEQ ID NO:3) and do not amplify bisulfite-untreated genomic DNA (data not shown). The amplified region corresponds to GenBank accession number AL355531, nt 46891-47156. PCR was carried out in a final volume of 30 μL, containing 6 pmol of each primer, 200 μM of each dNTP, 1.5 U HOT FIREPol DNA polymerase (Solis BioDyne, Tartu, Estonia) in buffer B (Solis BioDyne, Tartu, Estonia; delivered with HOT FIREPol DNA polymerase) containing 2.5 mM $MgCl_2$, and 2 μL of bisulfite-treated DNA as template. The initial denaturation (97 °C, 15 min) was followed by 37 cycles of 1 min at 95 °C, 1 min at 47.5 °C, 1 min at 72 °C, and a final extension step at 72 °C for 10 min.

PCR products were resolved on a 4% agarose gel, the specific band excised and purified with the QIAquick Gel Extraction System (Qiagen). The purified PCR products were cloned by using the TOPO TA Cloning Kit (Invitrogen) and at least 8 clones were subjected to the BigDye V.1.1 Cycle Sequencing chemistry (Applied Biosystems) and separated on a 3130 Genetic Analyzer (Applied Biosystems). Single clone sequences were analyzed with the BiQ Analyzer software *(25).*

1.4 COBRA

For amplification of bisulfite-treated DNA a two-step PCR approach was employed. The primers of the first step contained a non-matching M13 tail (nt 1 to 19 of SEQ ID NO:4 and nt 1 to 20 of SEQ ID NO:5; derived from bacteriophage M13, without binding site in the human genome). The second step used M13 primers labeled with FAM (6-Carboxyfluorescein) (fw) and JOE (2,7-dimethoxy-4,5-dichloro-6-carboxyfluorescein) (rev). The primers used for the first step were MGMT-CO-1 fw-5'-CAC GAC GTT GTA AAA CGA CGA TAT GTT GGG ATA GTT-3' (SEQ ID NO:4) and MGMT-CO-1 rev-5'-GGA TAA CAA TTT CAC ACA GGC CCA AAC ACT CAC CAA A-3' (SEQ ID NO:5). M13-primers used for the second step were MGMT-CO-2 fw-5'-(6-FAM)-CAC GAC GTT GTA AAA CGA C-3' (SEQ ID NO: 6) and MGMT-CO-2 rev-5'-(JOE)-GGA TAA CAA TTT CAC ACA GG-3' (SEQ ID NO:7). Amplification on bisulfite-untreated genomic DNA resulted in no product corresponding to the expected size (data not shown). The amplified region corresponds to GenBank accession number AL355531, nt 46892-46988. The first PCR was carried out in a final volume of 30 μL, containing 6 pmol of each primer, 200 μM of each dNTP, 1.5 U HOT FIREPol DNA polymerase (Solis BioDyne, Tartu, Estonia) in buffer B containing 2.5 mM $MgCl_2$, and 2 μL of bisulfite-treated DNA as template. The initial denaturation (97 °C, 15 min) was followed by 25 cycles of 1 min at 95 °C, 1 min at 48 °C, 1 min at 72 °C, and a final extension step at 72 °C for 10 min. The second PCR uses the same reaction setup except that 1 μL of the first PCR reaction was used as template. The initial denaturation (97 °C, 15 min) was followed by 25 cycles of 1 min at 95 °C, 1 min at 54 °C, 1 min at 72 °C, and a final extension step at 72 °C for 10 min.

PCR products were resolved on a 4% agarose gel, the specific bands excised and purified using the QIAquick Gel Extraction System (Qiagen). The elution was done with 50 μL $H_2O$. Subsequently the eluate was carefully evaporated with a Savant SC110 Speed Vac® Concentrator (Thermo Electron Corporation) and finally redissolved in 16 μL $H_3O$. Digestion of 3.5 μL of the purified PCR product was done by using the restriction endonucleases *Taq^a^I* and *BstUI* (New England Biolabs) in a final volume of 10 μL. The optimized reaction conditions for *Taq^a^I* are 5 U in NEBuffer 3 (New England BioLabs; 50 mM Tris-HCl, 10 mM $MgCl_2$, 100 mM NaCl, 1 mM dithiothreitol, pH 7.9 at 25 °C), 1× BSA (New England BioLabs; provided with the restriction endonucleases) and an incubation time of 4 h at 65 °C, whereas for *BstUI* 10 U were used in NEBuffer 2 and an incubation time of 16h at 60 °C. The solutions were mixed with 2x loading buffer (formamide : EDTA (25 mM) containing dextranblue as a marker) in a ratio 1:1 and 1.5 μL were loaded onto an ABI 377 (Applied Biosystems). The electropherograms were analyzed with the Gene Scan 3.1 software (Applied Biosystems). Methylation levels were calculated according to the following term: methylation [%] = ($AF_i$ / sum$AF_i$ + AND) x 100. $AF_i$ represents the integral of fragment $F_i$, sum$AF_i$ represents the sum of the integrals of all fragments and AND is the integral of the undigested product.

Each sample was analyzed in two separate PCR reactions using the same bisulfite preparation as template. Both amplification products were treated as described above and analyzed in duplicates.

1.5 SIRPH

Conditions used for generating the appropriate PCR product are identical to those described for bisulfite sequencing. 4 μL PCR product was treated with 1.6 μL ExoSAP-IT (GE Healthcare) at 37 °C for 15 min, heating at 80 °C for 15 min, and added to the primer extension mix. The primer used for primer extension reaction was 5'-GTG AGT GTT TGG GT-3' (SEQ ID NO: 10). The reaction was carried out in a final volume of 20 μL, containing 60 pmol primer, 100 μM each ddCTP and ddTTP, 1 U TermiPol (Solis BioDyne, Tartu, Estonia) in buffer C (Solis BioDyne, Tartu, Estonia; delvered with TermiPol) containing 5 mM $MgCl_2$. The initial denaturation (95 °C, 5 min) was followed by 50 cycles of 30 sec at 94 °C, 30 sec at 43 °C, and 1.5 min at 60 °C.

For the separation of the extended primers an aliquot of 19 μl of the SNuPE reaction was loaded on a dHPLC machine (WAVE DNA Fragment Analysis System from Transgenomics). The oven temperature was set to 50 °C and elution was done with a gradient of acetonitrile (20-40% over 15 min) made by mixing buffers A and

B, consisting of 0.1 M triethylammonium acetate (TEAA) buffer and 0.1 M TEAA buffer with 25% acetonitrile, respectively. The column was re-equilibrated by 90% buffer B for 1 min. The DNA was detected with a UV detector at 260 nm. Qualitative information for methylation is given as a ratio and calculated by the term: Q = (hC / hC + hT), where hC and hT represents the peak high of the signal contributed to the ddCTP-extended primer and the ddTTP-extended primer, respectively.

Each sample was analyzed in two separate PCR reactions using the same bisulfite preparation as template. Both amplification products were treated as described above and analyzed in duplicates.

1.6 Pyrosequencing™

The primers used for amplification of bisulfite-treated DNA were MGMT-Py fw-5'-Biotin-GGA TAT GTT GGG ATA GTT-3' (SEQ ID NO:2) and MGMT-Bis rev-5'-AAA CTA AAC AAC ACC TAA A-3' (SEQ ID NO:3) and do not amplify bisulfite-untreated genomic DNA (data not shown). PCR was carried out in a final volume of 50 μL, containing 10 pmol of each primer, 200 μM of each dNTP, 2.5 U HOT FIREPol DNA polymerase (Solis BioDyne, Tartu, Estonia) in buffer B (Solis BioDyne, Tartu, Estonia; delivered with HOT FIREPol DNA polymerase) containing 2.5 mM $MgCl_2$, and 3 μL of bisulfite-treated DNA as template. The initial denaturation (97 °C, 15 min) was followed by 38 cycles of 1 min at 95 °C, 1 min at 47.5 °C, 1 min at 72 °C, and a final extension step at 72 °C for 10 min.

40 μL of the PCR product was subjected to Pyrosequencing™. The primer used for primer extension reaction was 5'-CCC AAA CAC TCA CCA AA-3' (SEQ ID NO:8), which allows sequencing of the sequence: 5'-TCR CAA ACR ATA CRC ACC RC-3' (R = G or A)(SEQ ID NO:9). The sequencing reaction was performed on an automated PSQ 96MA System (Biotage) using the Pyro Gold Reagents Kit (Biotage). Purification and subsequent processing of the biotinylated single-strand DNA was done according to the manufacturers instructions. Resulting data was analyzed and quantified with the PSQ 96MA 2.1 software (Biotage).

Each tumor sample was analyzed in triplicates and each control sample in duplicates by individual PCR reactions using the same bisulfite preparation as template.

1.7 Statistical analysis

Statistical analysis was performed using the SPSS statistics package (SPSS for Windows, Chicago: SPSS Inc). Hierarchical clustering was based on the methylation averages and standard deviations of all CpG positions 1 to 25, calculated over all sequenced clones. Between-groups average linkage was used with squared Euclidean distance as interval measure. Logistic regression models were calculated with the WEKA package (26) using default parameters. For classification accuracy estimation leave-one-out cross-validation was performed as follows: for each of the 13 cases a logistic regression model was trained on the 12 other cases and tested on the selected case.

Example 2: Results

Aberrant methylation in the promoter region of *MGMT* has been associated with favorable outcome of cancer chemotherapy and is therefore a promising candidate marker for clinical treatment decisions *(15)*. We optimized this candidate epigenetic marker in a four-step process (Fig. 1), in order to translate it into a robust, reproducible and easy-to-use clinical marker. First, for an extensive candidate region we established full methylation patterns using bisulfite sequencing of DNA from 22 primary glioblastoma samples and three samples of normal brain tissue (white matter) as controls, and we clustered these into methylated and unmethylated cases. Second, we constructed candidate methylation markers from the sequence which can be readily analyzed by one of the following methods: COBRA, SIRPH, and Pyrosequencing™. Third, each epigenetic marker candidate was statistically evaluated on the full methylation profiles, and for each method optimal marker candidates were selected based on two criteria: strong correlation with overall promoter methylation and suitability/availability for testing by a given method. Fourth, all selected markers were validated on a subset of 14 out of 22 tumor samples with predefined methylation status for their experimental accuracy.

2.1 Methylation patterns of the *MGMT* promoter region in glioblastomas

In a first step we screened the genomic sequence corresponding to the promoter region and the first exon of the *MGMT* gene for CpG islands, using the CpG-Plot *(27)* web service (http://www.ebi.ac.uk/emboss/cpgplot/) with default parameters except for window = 200 and step = 10 (Fig. 2a). Next we designed non-discriminating bisulfite-specific primers (i.e. primers that do not contain CpGs as these are subject to methylation-dependent conversion) to amplify the region of interest from bisulfite-modified DNA. The amplicon includes all CpG positions analyzed via MSP by Hegi *et al. (15),* who used a nested MSP approach *(24)* with increased sensitivity to methylation compared to the original single step protocol *(9).* The location of their first step (1°) non-discriminating and second-step (2°) methylation-sensitive *(9)* primer pairs is for the unmethylated template nt 1060 to 1088 (fw) and nt 1125 to 1152 (rev) of SEQ ID NO: 1, and for the methylated template nt 1066 to 1088 (fw) and nt 1125 to 1146 of SEQ ID NO:1)(Fig. 2b). Our PCR product comprises 266 bp of DNA sequence and contains 27 CpG positions (Fig. 2c). CpGs 1 to 12 span the exon 1, CpGs 13 to 20 are intronic inside the predicted CpG island and the CpGs 21 to 27 are also intronic but outside the CpG island. The MSP used by Hegi *et al.* cover CpG positions 5 to 9 and 13 to 16 (Fig. 2b).

Bisulfite sequencing of 22 glioblastomas and three controls as described in the methods section resulted in reliable data for CpGs 1 to 25 (98% average conversion rate, 1.7% missing values), on which we focused our further analyses. We calculated methylation profiles for each sample, averaging over individual clones. Hierarchical clustering of the methylation profiles provides strong evidence for two distinct tumor subclasses (Fig. 3). The first subclass consists of tumor samples 01, 05, 06, 07 08, 09, 12, 13, 15, 16, 17, 19, 20, 23 and 25, as well as the largely unmethylated normal brain controls 1 to 3. The second group contains tumor samples 02, 03, 14, 18, 21, 22 and 24, which show significant methylation levels. Tumor 08 and 16, members of the first subclass, are best described as borderline cases. They exhibit few heavily methylated alleles among a large number of unmethylated alleles (see Fig. 6a and Fig. 6c, respectively). Therefore they deviate from the other tumor samples in the first subclass, which only exhibited sporadic methylation (e.g. Tumor 13, Fig. 6h) which is also observed among the control samples (e.g. Normal brain 3, Fig. 6j). This behavior is also apparent from the dendrogram where they are localized in between the clearly unmethylated and the clearly methylated samples. Based on these peculiarities and the fact that higher variation is present in the methylated subclass, we decided to regard these two intermediate cases as belonging to the methylated subclass for the purpose of methylation profile calculation. Consequently, tumor samples 01, 05, 06, 07, 09, 12, 13, 15, 17, 19, 20, 23 and 25 are classified as unmethylated samples and the corresponding methylation profile of this group (excluding the control samples) is shown in Figure 4, while samples 02, 03, 08, 14, 16, 18, 21, 22 and 24 are classified as methylated samples (Fig. 5).

Methylation profiles for CpG position 1 to 25 showed that the unmethylated tumor samples exhibit a median methylation of less than 10% (Fig. 4), while the methylated tumor samples exhibited a significant and variable level of methylation (Fig. 5). Figure 5 reveals that the median of the methylation level constantly increased from CpG 1 to 12 with values rising from 0% to 71% (exception: CpG 7). Beyond CpG 13 the methylation pattern becomes irregular and exhibits highly variable medians.

2.2 Construction of candidate markers for COBRA, SIRPH and Pyrosequencing™

Based on the observation that the CpGs at positions 3 to 13 represent stable indicators of the overall methylation level (with the exception of position 7 they all correlate with the overall methylation level with Pearson correlation coefficients above 0.85), we designed three bisulfite-based assays using experimental techniques that are suitable for clinical use to reliably detect the *MGMT* methylation levels in this region of interest. However, we note that CpG 13 is at a border line of methylated and unmethylated regions in both samples 16 (Fig. 6c) and 18 (Fig. 6d), indicating that this position may result in less representative measurements. For COBRA we selected a combination of two restriction endonucleases: *Taq*$^{\alpha}$*I* and *BstUI.* These enzymes assess DNA methylation at positions CpGs 1 and 2 simultaneously (methylation median in methylated samples: 0% and 17%), CpG 5 (methylation median in methylated samples: 38%), and CpGs 8 and 9 simultaneously (methylation median in methylated samples: 50% and 62%). Pyrosequencing™ covers CpGs 9 to 12 with median values in the range of 62% to 71% (in methylated samples). The single position CpG 13 (methylation median in methylated samples: 55%) was targeted for analysis by SIRPH. It is important to note that these different CpG sites will have different power of predicting *MGMT* methylation depending on the differences in methylation between the two tumor subclasses.

To evaluate these experimental methods we selected 14 out of the 22 GBMs, representing 8 unmethylated and 6 methylated tumor samples (43%). Two of these are highly methylated (sample 21, 22), three are moderately methylated (sample 14, 18, 24) and one is a borderline case (sample 16). Their methylation patterns obtained by bisulfite sequencing of single clones are shown in Fig. 6.

2.3 Statistical evaluation of candidate markers

For a suitable marker, we expect high correlation between the marker score and the overall promoter methylation. We pursued two routes to score the predictiveness of all marker candidates for the overall state of *MGMT* promoter methylation. First, based on the bisulfite sequencing data of all 22 tumor samples we calculated correlations between tumor promoter methylation subclass and each marker score or combination of marker scores that is experimentally feasible. Second, for the subset 14 tumor samples we experimentally re-analyzed all positions with the respective method and again calculated correlations between marker scores and the overall promoter methylation subclass as determined by bisulfite sequencing. Since little is known about the quantitative relationship between DNA methylation levels and gene silencing, we expect markers to reflect overall methylation states both in absolute terms (which can be measured by Pearson's correlation coefficient) and relative terms (which can be measured by the rank-based Spearman correlation coefficient). Furthermore, to enable comparison between markers that make use of different numbers of CpG positions, we focused our comparison on the mean methylation per marker candidate and did not perform any marker optimization at this stage. Most marker candidates showed strong correlation with overall methylation state (Table 1). For further analysis we picked the best and most strongly supported marker candidate for each method (IDs: COBRA: CO7 (5 CpG sites), SIRPH: SI01 (1 CpG site), and Pyrosequencing™: Py15 (4 CpG sites)(SEQ ID NO:9)), since they were expected to result in most robust detection of *MGMT* promoter methylation.

Table 1: Analysis of marker candidates. All but one correlation coefficient (0.433) are significantly different from zero (p<0.01 for each individual test).

| Exp. Method | Marker ID | Analyzed CpG positions | Correlation estimate from bisulfite data* | | Correlation estimate from new data** | |
|---|---|---|---|---|---|---|
| | | | Pearson's r | Spearman's rho | Pearson's r | Spearman's rho |
| COBRA | CO1 | 1-2 (comb.) | 0,696 | 0,554 | 0,433 | 0,670 |
| | CO2 | 8-9 (comb.) | 0,887 | 0,804 | 0,938 | 0,879 |
| | CO3 | 5 | 0,928 | 0,857 | 0,933 | 0,856 |
| | CO4 | 1-2 (comb.), 8-9 (comb.) | 0,927 | 0,881 | 0,916 | 0,875 |
| | CO5 | 1-2 (comb.), 5 | 0,937 | 0,869 | 0,926 | 0,857 |
| | CO6 | 8-9 (comb.), 5 | 0,949 | 0,908 | 0,947 | 0,856 |

(continued)

| Exp. Method | Marker ID | Analyzed CpG positions | Correlation estimate from bisulfite data* | | Correlation estimate from new data** | |
|---|---|---|---|---|---|---|
| | | | Pearson's r | Spearman's rho | Pearson's r | Spearman's rho |
| | CO7 | 1-2 (comb.), 8-9 (comb.), 5 | 0,961 | 0,929 | 0,942 | 0,856 |
| SIRPH | SI01 | 13 | 0,955 | 0,888 | 0,908 | 0,844 |
| Pyroseq. | Py01 | 9 | 0,877 | 0,734 | 0,901 | 0,838 |
| | Py02 | 10 | 0,894 | 0,817 | 0,886 | 0,792 |
| | Py03 | 11 | 0,962 | 0,898 | 0,867 | 0,740 |
| | Py04 | 12 | 0,959 | 0,910 | 0,872 | 0,825 |
| | Py05 | 9, 10 | 0,950 | 0,868 | 0,903 | 0,792 |
| | Py06 | 9, 11 | 0,935 | 0,858 | 0,886 | 0,774 |
| | Py07 | 9, 12 | 0,952 | 0,867 | 0,888 | 0,825 |
| | Py08 | 10, 11 | 0,954 | 0,886 | 0,895 | 0,748 |
| | Py09 | 10, 12 | 0,943 | 0,888 | 0,887 | 0,796 |
| | Py10 | 11, 12 | 0,967 | 0,898 | 0,873 | 0,758 |
| | Py11 | 9, 10, 11 | 0,958 | 0,872 | 0,898 | 0,787 |
| | Py12 | 9, 10, 12 | 0,962 | 0,874 | 0,895 | 0,792 |
| | Py13 | 9, 11, 12 | 0,957 | 0,863 | 0,883 | 0,793 |
| | Py14 | 10, 11, 12 | 0,961 | 0,887 | 0,888 | 0,769 |
| | Py15 | 9, 10, 11, 12 | 0,964 | 0,873 | 0,892 | 0,784 |
| | | | | | | |
| | | Min. | 0,696 | 0,554 | 0,433 | 0,670 |
| | | 1st quartile | 0,932 | 0,861 | 0,886 | 0,779 |
| | | Median | 0,952 | 0,873 | 0,895 | 0,793 |
| | | 3rd quartile | 0,960 | 0,888 | 0,912 | 0,850 |
| | | Max. | 0,967 | 0,929 | 0,947 | 0,879 |
| *calculated on all tumor samples, **calculated on tumor samples 12 to 25. | | | | | | |

2.4 COBRA

For fragment analysis we have chosen a system based on the restriction endonucleases *Taq*$^{\alpha}$*I* and *BstUI* with optimized reaction conditions (see Materials and Methods). Cleavage sites for *Taq*$^{\alpha}$*I* are between nt 1068 to 1069 and for *BstUI* nt 1045 and 1046, and nt 1087 to 1088 in the bisulfite-treated counterpart SEQ ID NO:1 (Fig. 2d). *Taq*$^{\alpha}$*I* has the recognition site TCGA, whereas *BstUI* cuts the site CGCG. The results obtained by analysis with *Taq*$^{\alpha}$*I* are quantitative because the analysis recognized only a single CpG position. The values given for *BstUI* are semi-quantitative because both neighboring CpG positions have to be methylated, resulting in the pattern CGCG, whereas a combination of a methylated and unmethylated status, appearing as the pattern CGTG or TGCG, is not recognized and therefore no signal observed. *Taq*$^{\alpha}$*I* cuts the appropriate pattern for CpG 5, while *BstUI* cut the appropriate pattern for CpG 1/2 and CpG 8/9.

To increase the sensitivity of the assay and to facilitate quantification, we used a two-step approach, which resulted in generation of a small PCR product double labeled with fluorescent dyes (Fig. 2d). The first step of the PCR reaction was performed using primers which contain M13 tails at their 5'-end. In the second round, M13 primers labeled with 6-FAM (forward primer) (SEQ ID NO:6) and JOE (reverse primer) (SEQ ID NO:7) were used to increase the detection limit of the PCR product and to increase the number of amplicons. The resulting PCR product consists of 136 bp (97 bp representing bisulfite converted DNA including CpG position 1 to 12 and 39 bp originated from the M13 primers). The values given for the analyzed samples were highly reproducible as demonstrated by the small standard deviation for each value (Table 2). For the unmethylated tumor samples and for the controls the values observed for the *Taq$^{\alpha}$I* site showed low levels of baseline methylation (<10%). The measurements exhibited only slight scattering around the calculated mean value. No restriction fragments were detectable after digestion with *BstUI.* The tumor samples classified as methylated showed fragments for both restriction endonucleases with values above the threshold. Only samples 14 and 18 did not show any detectable fragments for the first *BstUI* position (CpG1/2) but did so for the second one (CpG 8/9). In few cases (samples 16, 21, 22, and 24) the quantitative results obtained by *Taq$^{\alpha}$I* differed considerably from the values obtained by bisulfite sequencing of single clones.

2.5 SIRPH

The SIRPH approach is based on a primer extension reaction (SNuPE), where the primer was located directly in front of the CpG position to scan. Extension was performed by the appropriate dideoxynucleotides and separation of the extended primer products utilized dHPLC.

CpG 13 (Fig. 2c) is separated by 19 bases from CpG 12 and thus a SNuPE primer can be easily placed in front of CpG 13. A second reason to choose this position for this assay is its median value of methylation which is relatively high (55%) and therefore suitable for a predictive methylation marker. We observed that this position is not methylated or less methylated in samples 16 (Fig. 6c) and 18 (Fig. 6d), thus care should be taken when using of classification results based on only one CpG position. We decided to run the SIRPH assay only in a qualitative manner, resulting in the calculation of unit-free ratios. A qualitative SIRPH assay avoids the necessity of calibration curves and subsequent comparative determination of the methylation level, and the calculation of quotients could therefore be based on signals measured in a single run.

The normal brain controls and unmethylated tumor samples showed no or only a very faint signal for the ddCTP elongated primer (methylated position), whereas a high signal was observed with ddTTP (unmethylated position) (Fig. 7a). The methylated samples showed a significantly higher signal for the ddCTP extended primer (Fig. 7b). Each generated PCR product showed a high reproducibility for both values, while the values between two different PCR products sometimes differed. Strong variations were observed for the methylated tumor samples 14 and 18. Samples 16 and 20 showed a ratio of 0.12 which lies in between the quotients observed for the other unmethylated samples and those for methylated samples (Table 2). This may also explain the relatively high background noise observed for sample 20 using Pyrosequencing™ (Table 2) or bisulfite sequencing of single clones (Fig. 6i), while the quotient obtained for sample 16 was a result of the differing values measured for each PCR product (data not shown), reflecting the low methylation level for CpG 13 in this sample.

2.6 Pyroseguencing™

The region analyzed by Pyrosequencing™ (nt 1087 to 1106 of bisulfite-modified SEQ ID NO:1) includes CpGs 9 to 12 (Fig. 2c) which are individually assayed by this technique. The basic principle of Pyrosequencing™ is a primer extension reaction as for the SIRPH assay. However, by SIRPH we are directly measuring the final product while by Pyrosequencing™ it is possible to quantify the incorporation of different nucleotides by means of fluorescence, giving rise to a ratio at each position. The analysis of a CpG position can be interpreted as a Methyl-SNP, and therefore the ratio of C/TpG enables accurate measurement of the methylation level at this position.

The unmethylated tumor samples as well as the control samples showed ratios below 10% at all positions. Scattering around the mean value is very restricted, resulting in a small standard deviation (Table 2). Only sample 20 exhibited a slight increase in background noise of 5% to 7% for 3 out of 4 CpGs. This tendency was also observed for the CpG position interrogated by the SIRPH assay and by bisulfite sequencing results (Fig. 6i), indicating that this was not due to a technical artifact. The values for the methylated tumor samples 14, 16, 18 and 24 are in a good concordance with the results obtained by bisulfite sequencing. However, samples 21 and 22 demonstrated considerable lower methylation levels when compared to bisulfite sequencing data. Sample 21 exhibited differences in the range of 18% to 32% and relatively high values for the standard deviations (8% to 10%), whereas sample 22 showed differences between 29% to 36% with small standard deviations (1% to 5%). Nevertheless the general tendency of both results is consistent with high methylation levels in all cases. Typical pyrograms obtained by this method for an unmethylated and a methylated sample are shown in Figure 8.

To test accuracy and linearity for each individual CpG position interrogated by Pyrosequencing™, appropriate PCR templates were analyzed as serial mixtures of methylated and unmethylated products, with a 10% increment for methylation (Fig. 9). For each titration curve linearity was observed and the measured data points were in good agreement with the theoretical curve (straight line) The arrangement of the data points given for CpG 9, CpG 10 and CpG 12 shared the same shape. A moderate underestimation for the completely methylated PCR product was observed (91%, 92%, and 95%, respectively). The titration curve for CpG 11 showed minor but consistent overestimation in the range of 5% to 7% in between the methylation level of 10% to 90%, but reflected accurately the values expected for the completely unmethylated and the fully methylated PCR product.

Table 2: Experimental data obtained by bisulfite sequencing of single clones (Bis-S.) for selected CpG positions, Pyrosequencing™ (Pyro.), COBRA, and SIRPH (Q-values are given for SIRPH). The values are given by Mean ± SD.

| Sample | Method | CpG 01 [%] | CpG 02 [%] | CpG 05 [%] | CpG 08 [%] | CpG 09 [%] | CpG 10 [%] | CpG 11 [%] | CpG 12 [%] | CpG 13 [%] |
|---|---|---|---|---|---|---|---|---|---|---|
| Tu12 | Bis-S. | 0 ± 0 | 0 ± 0 | 0 ± 0 | 0 ± 0 | 11 ± 31 | 11 ± 31 | 0 ± 0 | 0 ± 0 | 0 ± 0 |
| | Pyro. | | | | | 0 ± 0 | 2 ± 3 | 0 ± 0 | 0 ± 0 | |
| | COBRA | 0 ± 0 | 0 ± 0 | 3 ± 2 | 0 ± 0 | 0 ± 0 | | | | |
| | SIRPH | | | | | | | | | 0±0 |
| Tu13 | Bis-S. | 0 ± 0 | 0 ± 0 | 0 ± 0 | 0 ± 0 | 0 ± 0 | 0 ± 0 | 0 ± 0 | 0 ± 0 | 0 ± 0 |
| | Pyro. | | | | | 2 ± 3 | 4 ± 3 | 4 ± 3 | 5 ± 3 | |
| | COBRA | 0 ± 0 | 0 ± 0 | 5 ± 3 | 0 ± 0 | 0 ± 0 | | | | |
| | SIRPH | | | | | | | | | 0.08 ± 0.01 |
| Tu14 | Bis-S. | 0 ± 0 | 8 ± 27 | 15 ± 36 | 54 ± 50 | 62 ± 49 | 62 ± 49 | 62 ± 49 | 54 ± 50 | 46 ± 50 |
| | Pyro. | | | | | 56 ± 4 | 57 ± 7 | 59 ± 3 | 62 ± 3 | |
| | COBRA | 0 ± 0 | 0 ± 0 | 27 ± 6 | 25 ± 3 | 25 ± 3 | | | | |
| | SIRPH | | | | | | | | | 0.23 ± 0 .05 |
| Tu15 | Bis-S. | 0 ± 0 | 0 ± 0 | 0 ± 0 | 0 ± 0 | 0 ± 0 | 0 ± 0 | 0 ± 0 | 0 ± 0 | 0 ± 0 |
| | Pyro. | | | | | 2 ± 3 | 3 ± 4 | 3 ± 4 | 2 ± 4 | |
| | COBRA | 0 ± 0 | 0 ± 0 | 7 ± 3 | 0 ± 0 | 0 ± 0 | | | | |
| | SIRPH | | | | | | | | | 0.02 ± 0.03 |
| Tu16 | Bis-S. | 0 ± 0 | 0 ± 0 | 0 ± 0 | 25 ± 43 | 25 ± 43 | 0 ± 0 | 25 ± 43 | 25 ± 43 | 0 ± 0 |
| | Pyro. | | | | | 23 ± 3 | 19 ± 1 | 21 ± 4 | 26 ± 3 | |
| | COBRA | 12 ± 3 | 12 ± 3 | 18 ± 6 | 17 ± 1 | 17 ± 1 | | | | |
| | SIRPH | | | | | | | | | 0.27 ± 0.02 |
| Tu17 | Bis-S. | 0 ± 0 | 0 ± 0 | 0 ± 0 | 0 ± 0 | 0 ± 0 | 13 ± 33 | 0 ± 0 | 0 ± 0 | 13 ± 33 |
| | Pyro. | | | | | 2 ± 4 | 3 ± 4 | 3 ± 5 | 2 ± 4 | |
| | COBRA | 0 ± 0 | 0 ± 0 | 4 ± 2 | 0 ± 0 | 0± 0 | | | | |
| | SIRPH | | | | | | | | | 0.03 ± 0.01 |
| Tu18 | Bis-S. | 15 ± 36 | 15 ± 36 | 57 ± 50 | 50 ± 50 | 57 ± 50 | 57 ± 50 | 57 ± 50 | 57 ± 50 | 21 ± 41 |
| | Pyro. | | | | | 51 ± 1 | 49 ± 1 | 54 ± 3 | 49 ± 1 | |
| | COBRA | 0 ± 0 | 0 ± 0 | 40 ± 7 | 31 ± 6 | 31 ± 6 | | | | |
| | SIRPH | | | | | | | | | 0.12 ± 0.04 |
| Tu19 | Bis-S. | 0 ± 0 | 0 ± 0 | 0 ± 0 | 0 ± 0 | 0 ± 0 | 0 ± 0 | 0 ± 0 | 0 ± 0 | 0 ± 0 |
| | Pyro. | | | | | 0 ± 0 | 2 ± 4 | 6 ± 9 | 0 ± 0 | |
| | COBRA | 0 ± 0 | 0 ± 0 | 3 ± 3 | 0 ± 0 | 0 ± 0 | | | | |
| | SIRPH | | | | | | | | | 0.03 ± 0.02 |

(continued)

| Sample | Method | CpG 01 [%] | CpG 02 [%] | CpG 05 [%] | CpG 08 [%] | CpG 09 [%] | CpG 10 [%] | CpG 11 [%] | CpG 12 [%] | CpG 13 [%] |
|---|---|---|---|---|---|---|---|---|---|---|
| Tu20 | Bis-S. | 0 ± 0 | 0 ± 0 | 0 ± 0 | 0 ± 0 | 0 ± 0 | 0 ± 0 | 0 ± 0 | 0 ± 0 | 0 ± 0 |
| | Pyro. | | | | | 2 ± 3 | 7 ± 3 | 5 ± 3 | 7 ± 3 | |
| | COBRA | 0 ± 0 | 0 ± 0 | 6 ± 3 | 0 ± 0 | 0 ± 0 | | | | |
| | SIRPH | | | | | | | | | 0.12 ± 0.01 |
| Tu21 | Bis-S. | 60 ± 49 | 40 ± 49 | 82 ± 39 | 92 ± 28 | 83 ± 37 | 83 ± 37 | 92 ± 28 | 75 ± 43 | 83 ± 37 |
| | Pyro. | | | | | 59 ± 8 | 53 ± 9 | 60 ± 10 | 57 ± 8 | |
| | COBRA | 7 ± 2 | 7 ± 2 | 47 ± 8 | 47 ± 7 | 47 ± 7 | | | | |
| | SIRPH | | | | | | | | | 0.46 ± 0.03 |
| Tu22 | Bis-S. | 17 ± 37 | 67 ± 47 | 85 ± 36 | 86 ± 35 | 86 ± 35 | 86 ± 35 | 86 ± 35 | 86 ± 35 | 83 ± 37 |
| | Pyro. | | | | | 53 ± 2 | 50 ± 1 | 57 ± 4 | 50 ± 5 | |
| | COBRA | 3 ± 1 | 3 ± 1 | 37 ± 4 | 30 ± 3 | 30 ± 3 | | | | |
| | SIRPH | | | | | | | | | 0.43 ± 0.01 |
| Tu23 | Bis-S. | 0 ± 0 | 0 ± 0 | 0 ± 0 | 0 ± 0 | 11 ± 31 | 0 ± 0 | 0 ± 0 | 0 ± 0 | 0 ± 0 |
| | Pyro. | | | | | 0 ± 0 | 0 ± 0 | 0 ± 0 | 0 ± 0 | |
| | COBRA | 0 ± 0 | 0 ± 0 | 4 ± 2 | 0 ± 0 | 0 ± 0 | | | | |
| | SIRPH | | | | | | | | | 0.04 ± 0.01 |
| Tu24 | Bis-S. | 0 ± 0 | 33 ± 47 | 73 ± 45 | 36 ± 48 | 64 ± 48 | 46 ± 50 | 73 ± 45 | 73 ± 45 | 55 ± 50 |
| | Pyro. | | | | | 64 ± 3 | 35 ± 7 | 86 ± 4 | 64 ± 4 | |
| | COBRA | 3 ± 0 | 3 ± 0 | 44 ± 5 | 22 ± 2 | 22 ± 2 | | | | |
| | SIRPH | | | | | | | | | 0.44 ± 0.03 |
| Tu25 | Bis-S. | 0 ± 0 | 0 ± 0 | 0 ± 0 | 0 ± 0 | 0 ± 0 | 0 ± 0 | 10 ± 30 | 0 ± 0 | 0 ± 0 |
| | Pyro. | | | | | 0 ± 0 | 0 ± 0 | 0 ± 0 | 0 ± 0 | |
| | COBRA | 0 ± 0 | 0 ± 0 | 5 ± 4 | 0 ± 0 | 0 ± 0 | | | | |
| | SIRPH | | | | | | | | | 0.03 ± 0.01 |
| NB01 | Bis-S. | 10 ± 30 | 0 ± 0 | 10 ± 30 | 10 ± 30 | 20 ± 40 | 10 ± 30 | 20 ± 40 | 10 ± 30 | 0 ± 0 |
| | Pyro. | | | | | 0 ± 0 | 0 ± 0 | 0 ± 0 | 0 ± 0 | |
| | COBRA | 0 ± 0 | 0 ± 0 | 5 ± 4 | 0 ± 0 | 0 ± 0 | | | | |
| | SIRPH | | | | | | | | | 0.03 ± 0.01 |
| NB02 | Bis-S. | 0 ± 0 | 0 ± 0 | 0 ± 0 | 0 ± 0 | 0 ± 0 | 8 ± 28 | 0 ± 0 | 0 ± 0 | 0 ± 0 |
| | Pyro. | | | | | 0 ± 0 | 3 ± 2 | 0 ± 0 | 3 ± 3 | |
| | COBRA | 0 ± 0 | 0 ± 0 | 4 ± 2 | 0 ± 0 | 0 ± 0 | | | | |
| | SIRPH | | | | | | | | | 0.06 ± 0.00 |
| NB03 | Bis-S. | 0 ± 0 | 0 ± 0 | 0 ± 0 | 0 ± 0 | 0 ± 0 | 0 ± 0 | 0 ± 0 | 0 ± 0 | 0 ± 0 |
| | Pyro. | | | | | 0 ± 0 | 0 ± 0 | 0 ± 0 | 3 ± 2 | |
| | COBRA | 0 ± 0 | 0 ± 0 | 3 ± 3 | 0 ± 0 | 0 ± 0 | | | | |
| | SIRPH | | | | | | | | | 0.05 ± 0.00 |

2.7 Statistical correlation and validation of candidate marker regions

The experimental analyses reported in the previous section indicate that all selected candidate regions of the *MGMT* promoter could discriminate between the unmethylated and the methylated subclass, but provided little information on their relative performance compared to each other, their robustness towards intermediate cases and the optimal separation between the two subclasses. In this section we compared the most accurate CpG marker for each method on the validation dataset (tumor samples 12 to 25) after optimization by logistic regression. Furthermore, we determined optimal classification boundaries between methylated and unmethylated cases. Since sample 16 showed intermediate behavior for all technologies and may constitute an outlier, we excluded it from the training phase.

For both CO7 (COBRA, 5 CpG sites) and the Py15 (Pyrosequencing™, 4 CpG sites) marker candidate, logistic regression led to correct classification of all 13 tumor samples (100% test set accuracy as determined by leave-one-out cross-validation). For the SI01 (SIRPH, 1 CpG site) marker, 12 out of 13 tumor samples were classified correctly (92% test set accuracy). This result was consistent with our observation that all marker candidates perform well, but showed that the SIRPH marker was less robust than the other two markers. This reflected the differences in the positions of the CpGs used in each marker and the number of analysed CpGs (being only one CpG for the SIRPH marker). SIRPH can only be used if at least 6 bases adjacent to a critical CpG site do not contain a CpG position. For the region of interest, namely nt 1087 to 1106 of SEQ ID NO:1 this is problematic, as it contains CpG positions at at least every 6$^{th}$ position.

Next, we used logistic regression on the full validation dataset (again excluding tumor 16) to calculate optimal separation between unmethylated and methylated tumors, resulting in the following three classification formulae:

$$\text{COBRA: } Score_{CO7} = -339.385 \cdot CpG_{1/2} + 196.192 \cdot CpG_{8/9} + 137.296 \cdot CpG_5 - 21.803\,;$$

$$\text{SIRPH: } Score_{SI01} = 306.601 \cdot CpG_{13} - 36.792\,;$$

Pyrosequencing™:

$$Score_{Py15} = 21.330 \cdot CpG_9 + 24.806 \cdot CpG_{10} + 18.637 \cdot CpG_{11} + 21.503 \cdot CpG_{12} - 20.197\,,$$

where CpG variables refer to the measured methylation score at each position. Overall positive scores predict promoter methylation, negative scores the absence of promoter methylation. For COBRA, we observed an unexpected negative coefficient at CpG position 1/2, indicating that a high methylation level at this position was not a good predictor of a high overall methylation level.

For all formulae, the resulting score for any single tumor not only gives a prediction of the overall promoter methylation state but also provides a confidence estimate: the further the score deviates from zero, the safer is the prediction. In order to distinguish unambiguous regions from intermediate regions, we re-applied these formulae to the full validation dataset, now including the borderline case 16 (Fig. 10).

For CO7 we observed that scores below -15 were highly indicative of overall absence of methylation, while scores above 40 were consistently associated with overall promoter methylation. Between these safety margins, no clear conclusion was possible. For SI01, the safely unmethylated region started below -10, while the safely methylated region started above 70. Separation was generally low due to high score variance within subclasses. For Py15, safe prediction of unmethylated promoter state was possible below -10, while methylation could be predicted above 10. Due to low score variance within subclasses, large separation margins were observed. Fig 10 visually confirmed our previous observation that candidate marker Py15 used for Pyrosequencing™ is superior to both CO7 and SI01. The accessibility of the CpG positions 9 to 12 by Pyrosequencing™, which reflected more accurately the overall methylation status than the other CpGs studied by COBRA and SIRPH, put the Pyrosequencing™ approach in a clear advantage over the other two approaches.

Since Py15 consists of four marker positions it is more tolerant towards noise than the other markers. Simulation showed that flipping any one position to zero or to one (which can theoretically happen due to rare C-T-SNPs at the analyzed CpG dinucleotide) can only convert a clearly methylated / unmethylated sample into a doubtful prediction or vice versa, but cannot - for none of the samples we analyzed - convert a clear methylated case into a clear unmethylated case or vice versa. Therefore, we conclude that the Py15 marker and, to a lesser extent, the CO7 marker as described here provide the necessary statistical robustness to cope with doubtful and intermediate cases.

**References**

1. Ludlum DB. Mutat. Res. 1990; 233:117-26.

2. Pegg AE. Mutat. Res. 2000;462:83-100.
3. Pegg AE, Dolan ME, Moschel RC. Prog Nucleic Acid Res Mol Biol 1995;51:167-223.
4. Wibley JEA, Pegg AE, Moody PCE. Nucl Acids Res 2000;28:393-401.
5. Karran P, Bignami M. Bioessays 1994;16:833-39.
6. Margison GP, Povey AC, Kaina B, Santibáñez Koref MF. Carcinogenesis 2003;4:625-35.
7. Citron M et al. Cancer Res 1991;51:4131-34.
8. Silber JR, Mueller BA, Ewers TG, Berger MS. Cancer Res 1993;53:3416-20.
9. Esteller M, Hamilton SR, Burger PC, Baylin SB, Herman, JG. Cancer Res 1999;59:793-97.
10. Fritz G, Tano K, Mitra S, Kaina B. Mol Cell Biol 1991;11:4660-68.
11. Grombacher T, Mitra S, Kaina B. Carninogenesis 1996;17:2329-36.
12. Esteller M et al. N Engl J Med 2000;343:1350-54.
13. Paz MF et al. Clin Cancer Res 2004;10:4933-38.
14. Gerson SL. Nat Rev Cancer 2004;4:296-307.
15. Hegi ME et al. N Engl J Med 2005;352:997-1003.
16. Roos WP et al. Oncogene advance online publication 3 July 2006, (DOI: 10.1038/sj.onc.1209785).
17. Herman JG, Graff JR, Myöhänen S, Nelkin BD, Baylin SB. Proc. Natl Acad Sci USA 1986; 93: 9821-26.
18. Derks S, Lentjes, MHFM, Hellebrekers, DMEI, de Bruïne, AP, Herman, JG, van Engeland, M. Cell Oncol 2004; 26:291-99.
19. Xiong Z, Laird P. W. Nucl Acids Res 1997;25:2532-34.
20. El-Maarri O, Herbiniaux U, Walter J, Oldenburg J. Nucl Acids Res 2002;30:e25.
21. Tost J, Dunker J, Gut IG. Biotechniques 2003;35: 152-56.
22. Kleihues P, Webster KC. Pathology and Genetics of Tumours of the Nervous System. World Health Organization Classification of Tumours, 2nd edn. Lyon: Oxford University Press 2000.
23. Sambrook J, Fritsch EF, Maniatis T. Molecular cloning. Cold Spring Harbor, NY: Cold Spring Harbor Laboratory Press 1989.
24. Palmisano WA et al. Cancer Res 2000;60:5954-58.
25. Bock C, Reither S, Mikeska T, Paulsen M, Walter J, Lengauer T. Bioinformatics 2005;21:4067-68.
26. Witten IH, Frank E. Data mining: practical machine learning tools and techniques with Java implementations, xxv. Morgan Kaufmann, San Francisco, Calif., 2000:371p.
27. Larsen F, Gundersen G, Lopez R, Prydz H. Genomics 1992;13:1095-107.
28. Shaw RJ, Akufo-Tetteh EK., Risk JM., Field JK, Liloglou T. Nucl Acids Res 2006;34:e78.

## Claims

**1.** A method for determination of the methylation degree of the $O^6$-methylguanine DNA methyltransferase gene (*MGMT*) 5' non-coding region ("*MGMT* promoter") in a DNA sample, which comprises

(a) treating the DNA sample with bisulfite;
(b) amplifying a segment ("amplified segment") of the bisulfite-modified DNA ("bisulfite-treated counterpart") obtained in step (a) by PCR using the bisufite treated DNA as template, wherein the amplified segment comprises at least 20 consecutive bisulfite-modified nucleotides within the region nt 1041 to 1260 of the native *MGMT* sequence represented by SEQ ID NO: and spans at least three CpG sites ("selected CpG sites"); and
(c) analysing the amplification product and determining the methylation status of the selected CpG sites in the amplified segment.

**2.** The method of claim 1, wherein

(i) the amplified segment in step (b) spans at least 4 selected CpG sites, preferably the at least three selected CpG sites covered by the amplified segment are selected from the group consisting of the CpG sites at positions nt 1044+1045 (CpG 1), nt 1046+1047 (CpG 2), nt 1057+1058 (CpG 3), nt 1064+1065 (CpG 4), nt 1069+1070 (CpG 5), nt 1072+1073 (CpG 6), nt 1076+1077 (CpG 7), nt 1086+1087 (CpG 8), nt 1088+1089 (CpG 9), nt 1093+1094 (CpG 10), nt 1098+1099 (CpG 11), nt 1104+1105 (CpG 12), and nt 1125+1126 (CpG 13) in the native *MGMT* DNA sequence represented by SEQ ID NO: 1; and/or
(ii) the amplified segment comprises at least 20 consecutive nucleotides of the bisulfite-treated counterpart of the sequence represented by nt 1041 to 1105 of SEQ ID NO:1 and preferably comprises the bisulfite-treated counterpart of the seqeunce represented by nt 1041 to 1089 or 1086 to 1105 of SEQ ID NO: 1; and/or
(iii) the primer pair used in the amplification step (b) consists of a forward and reverse primer, wherein the forward primer is complementary to at least 10 consecutive nucleotides of nt 806 to 822, 931 to 954, 1026 to 1043, or 1105 to 1124 of SEQ ID NO:1 or its bisulfite-treated counterpart, and the reverse primer is complementary to at least 10 consecutive nt of nt 1105 to 1124, nt 1250 to 1296, 1298 to 1323, 1325 to 1345, 1361 to 1401, 1410 to 1447 or 1449 to 1482 of SEQ ID NO:1 or its bisulfite-treated counterpart, and wherein preferably at least one of the primers is a primer complementary to said segments of SEQ ID NO:1 after the bisulfite modification.

**3.** The method of claim 2, wherein the at least three selected CpG sites covered by the amplified segment are selected from the group consisting of CpG 3 to 13, preferably of CpG 5 to 12, more preferably of CpG 9 to 11.

**4.** The method of claim 3, wherein the selected CpG sites covered by the amplified fragment comprise either (i) CpG 9 to CpG 12 or (ii) CpG 5, 8 and 9.

**5.** The method of any one of claims 1 to 4, wherein step (c) comprises Pyrosequencing™ or restriction analysis of the amplification product.

**6.** The method of claim any one of claims 1 to 5, wherein
step (b) comprises only one PCR amplification reaction;

the analysis step (c) is performed using Pyrosequencing; and
the selected CpG sites comprise at least three CpG sites selected from CpG 9 to 12, and more preferably comprise all 4 CpG sites CpG 9 to 12.

**7.** The method of claim 6, wherein

(i) in the primer pair used in the amplification step (b) the forward primer is complementary to at least 10 consecutive nucleotides of nt 1026 to 1043 of SEQ ID NO:1 or its bisulfite-treated counterpart, and the reverse primer is complementary to at least 10 consecutive nt of nt 1250 to 1296, preferably of nt 1260 to 1296 of SEQ ID NO:1 or its bisulfite-treated counterpart; and/or
(ii) in step (c) a segment comprising at least nt 1088 to 1098 of SEQ ID NO:1, or the reverse strand of said segment is pyrosequenced, preferably using a primer which is complementary to at least 10 nt downstream of said segment.

**8.** The method of any one of claims 1 to 5, wherein
the analysis step (c) comprises a restriction digestion of the amplification product and subsequent analysis of the restriction products; and
the selected CpG sites comprise at least CpG 5, 8 and 9, preferably comprise CpG 1, 2, 5, 8 and 9.

**9.** The method of claim 8, wherein

(i) step (b) comprises two PCR reactions, a first PCR reaction with a first primer pair using the template as defined in claim 1, and a second PCR reaction with a second primer pair using the amplification product of the first PCR reaction and a second primer pair, wherein the first primer pair contains 5'-tails of at least 10 nt length which are identical to the second primer sequences, and in the second primer pair one primer is labelled at its 5'tail with one dye molecule and the second primer is labelled at its 5'tail with a different dye molecule; and
(ii) step (c) is performed on the amplification product of the second PCR reaction.

**10.** The method of claim 8 or 9, wherein

(i) in the primer pair used in the first PCR in amplification step (b) the forward primer is complementary to at least 10 consecutive nucleotides of nt 1026 to 1043 of SEQ ID NO:1 or its bisulfite-treated counterpart, and the reverse primer is complementary to at least 10 consecutive nt of nt 1105 to 1124, nt 1250 to 1296, 1298 to 1323, 1325 to 1345, 1361 to 1401, 1410 to 1447 or 1449 to 1482, preferably of nt 1105 to 1124 of SEQ ID NO: 1 or its bisulfite-treated counterpart;

(ii) the primers of the primer pair used in the second PCR are at least 13 nt long and are labeled at their 5' ends with different fluorescent or luminescent dye molecules;

(iii) the amplification product which is analyzed in step (c) is labelled with two dye molecules which are different from each other, wherein one dye molecule is covalently linked to the 3'end and the other to the 5'end of the amplification product, and wherein preferably said amplification product is the result of the second PCR in step (b) using the labelled primers as defined in claim 10; and/or

(iv) at least one restriction endonuclease used in the restriction digestion of step (c) has a recognition site which spans at least one of the selected CpG sites and does recognize only a recognition site containing CpG at the CpG site in the amplification product obtained by step (b).

**11.** The method of any one of claims 1 to 10, wherein the DNA sample is

(i) genomic DNA; and/or

(ii) isolated from mammalian, preferably from human tissue, and wherein said tissue is preferably a kryopreserved or formalin-fixed tissue; and/or

(iii) isolated from a tumor tissue, preferably tumor tissue isolated from glioma, diffuse large B-cell lymphoma (DLBLC), colorectal carcinoma, non-small cell lung carcinoma, lymphoma, head and neck carcinoma, brain tumors, more preferably tumor tissue isolated from glioma, colorectal carcinoma and DLBLC, most preferably tumor tissue isolated from malignant glioma, especially glioblastoma, and DLBLC.

**12.** A method for prediction of the responsiveness of tumors to alkylating agents which comprises performing the method of any one of claims 1 to 11, wherein the methylation degree of the selected CpG sites is indicative for the responsiveness of the tumor.

**13.** The method of claim 12, wherein a methylation degree of 75 % or more of the selected CpG sites indicates that the tumor is susceptible to alkylating agents.

**14.** A method for diagnosis or prognosis of a cancer which comprises performing the method of any one of claims 1 to 11, wherein the methylation degree of the selected CpG sites is indicative for a cancer or cancer risk.

**15.** An isolated DNA molecule which is

(i) the amplification product as obtainable by step (b) according to any one of claims 1 to 11;

(ii) a primer for the PCR reaction in step (b) as defined in claim 2 (iii), preferably as defined in claims 7(i), 9 (i), 10 (i) or 10 (ii); or

(iii) a primer for the pyrosequencing reaction as defined in claim 7 (ii).

**16.** A kit for performing the method of any one of claims 1 to 14, which comprises one or more of the primers as defined in claim 15 and/or one or more of the restriction endonucleases as defined in claim 10 (iv).

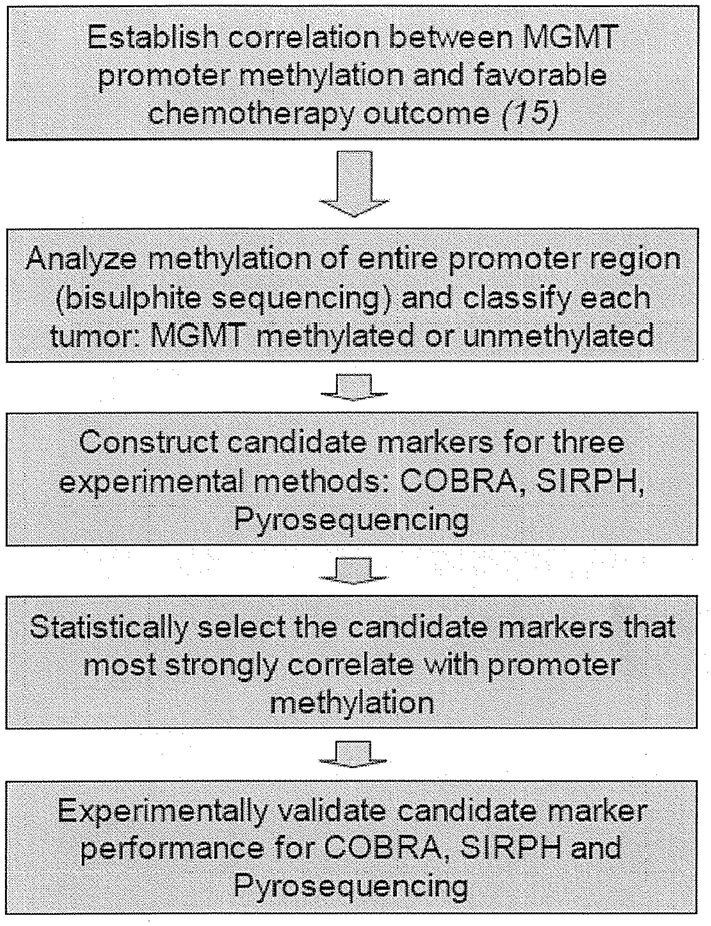
Establish correlation between MGMT promoter methylation and favorable chemotherapy outcome (15)
Analyze methylation of entire promoter region (bisulphite sequencing) and classify each tumor: MGMT methylated or unmethylated
Construct candidate markers for three experimental methods: COBRA, SIRPH, Pyrosequencing
Statistically select the candidate markers that most strongly correlate with promoter methylation
Experimentally validate candidate marker performance for COBRA, SIRPH and Pyrosequencing

**Fig.1**

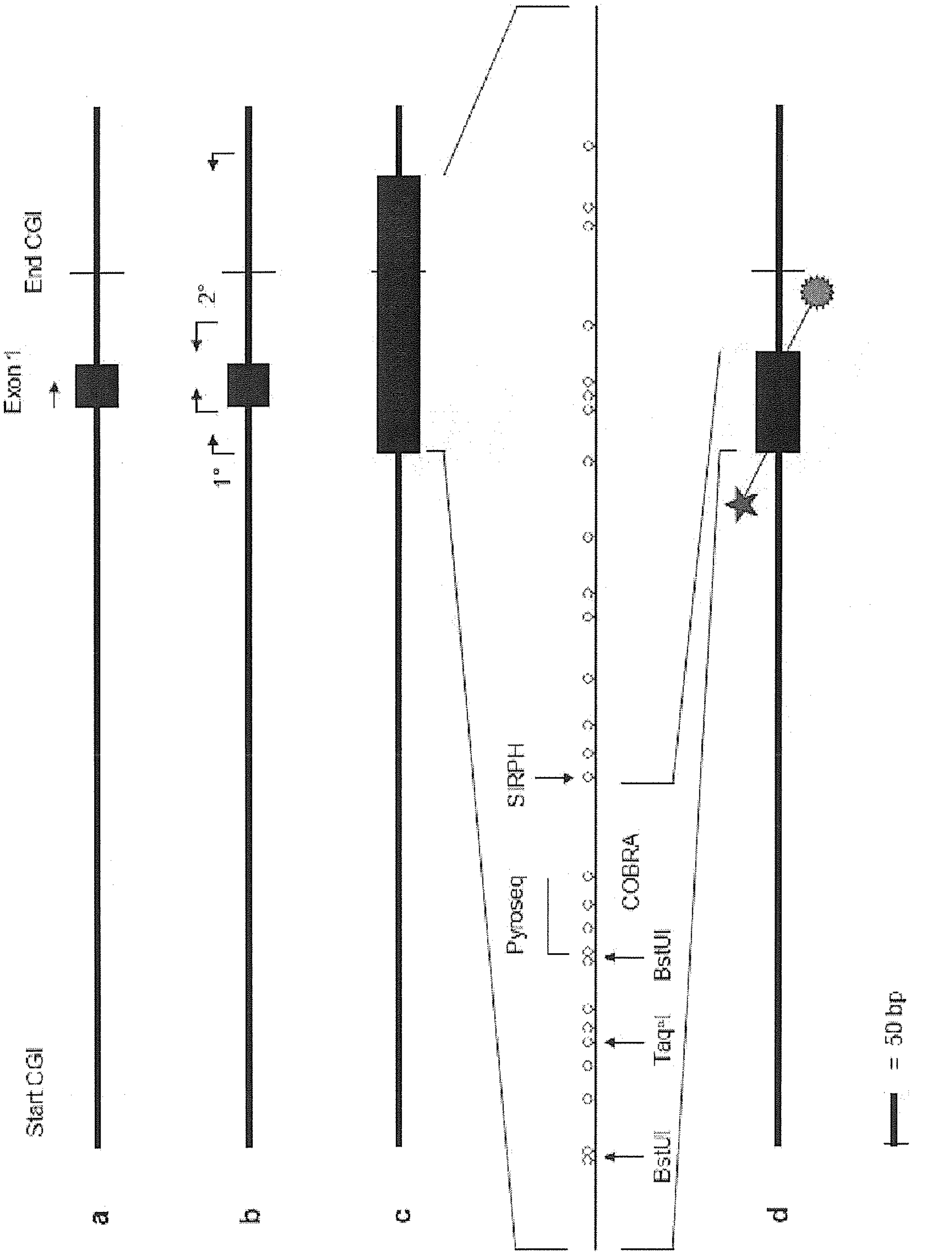
Start CGI
Exon 1
End CGI
a
b
c
d
1°
2°
BstUI
TaqI
BstUI
COBRA
Pyroseq
SIRPH
= 50 bp

Fig.2

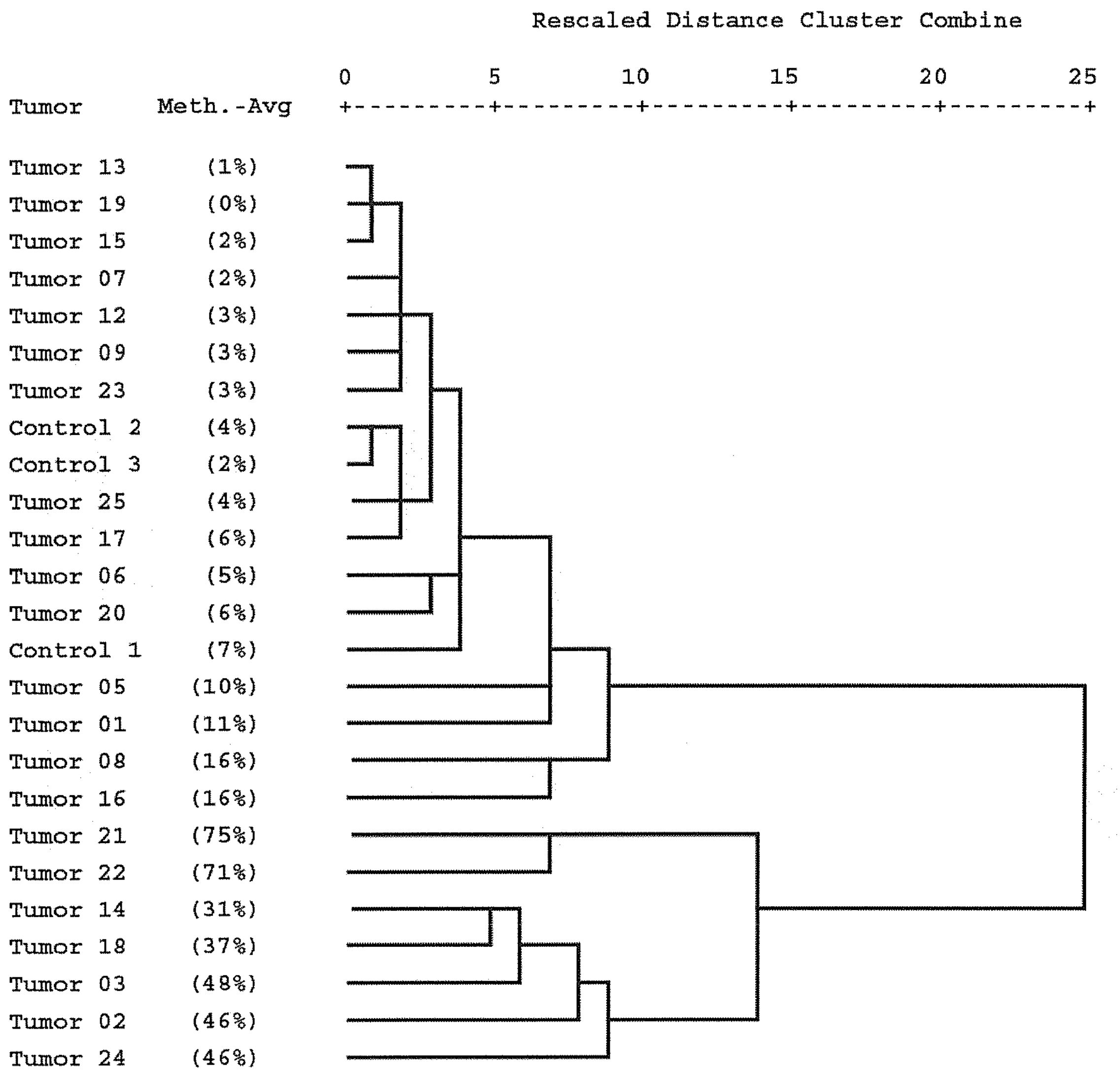
Rescaled Distance Cluster Combine
0
5
10
15
20
25
Tumor
Meth.-Avg
Tumor 13 (1%)
Tumor 19 (0%)
Tumor 15 (2%)
Tumor 07 (2%)
Tumor 12 (3%)
Tumor 09 (3%)
Tumor 23 (3%)
Control 2 (4%)
Control 3 (2%)
Tumor 25 (4%)
Tumor 17 (6%)
Tumor 06 (5%)
Tumor 20 (6%)
Control 1 (7%)
Tumor 05 (10%)
Tumor 01 (11%)
Tumor 08 (16%)
Tumor 16 (16%)
Tumor 21 (75%)
Tumor 22 (71%)
Tumor 14 (31%)
Tumor 18 (37%)
Tumor 03 (48%)
Tumor 02 (46%)
Tumor 24 (46%)

**Fig.3**

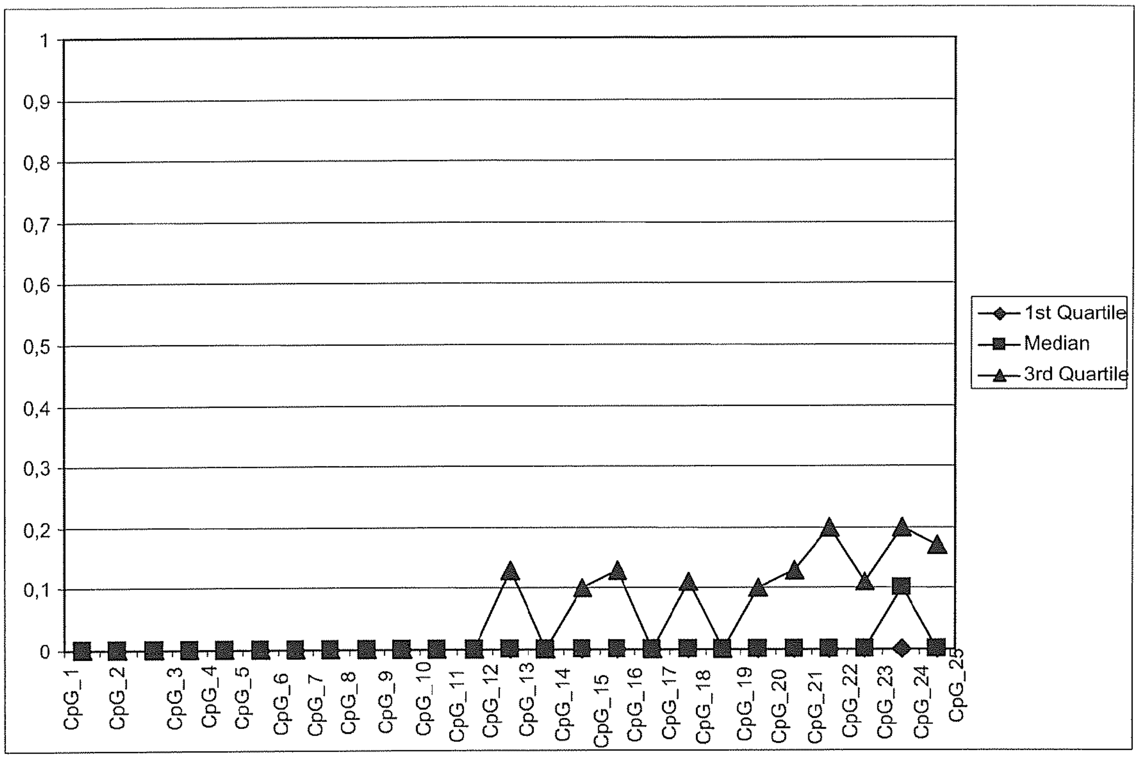
1
0,9
0,8
0,7
0,6
0,5
0,4
0,3
0,2
0,1
0
CpG_1
CpG_2
CpG_3
CpG_4
CpG_5
CpG_6
CpG_7
CpG_8
CpG_9
CpG_10
CpG_11
CpG_12
CpG_13
CpG_14
CpG_15
CpG_16
CpG_17
CpG_18
CpG_19
CpG_20
CpG_21
CpG_22
CpG_23
CpG_24
CpG_25
1st Quartile
Median
3rd Quartile

Fig.4

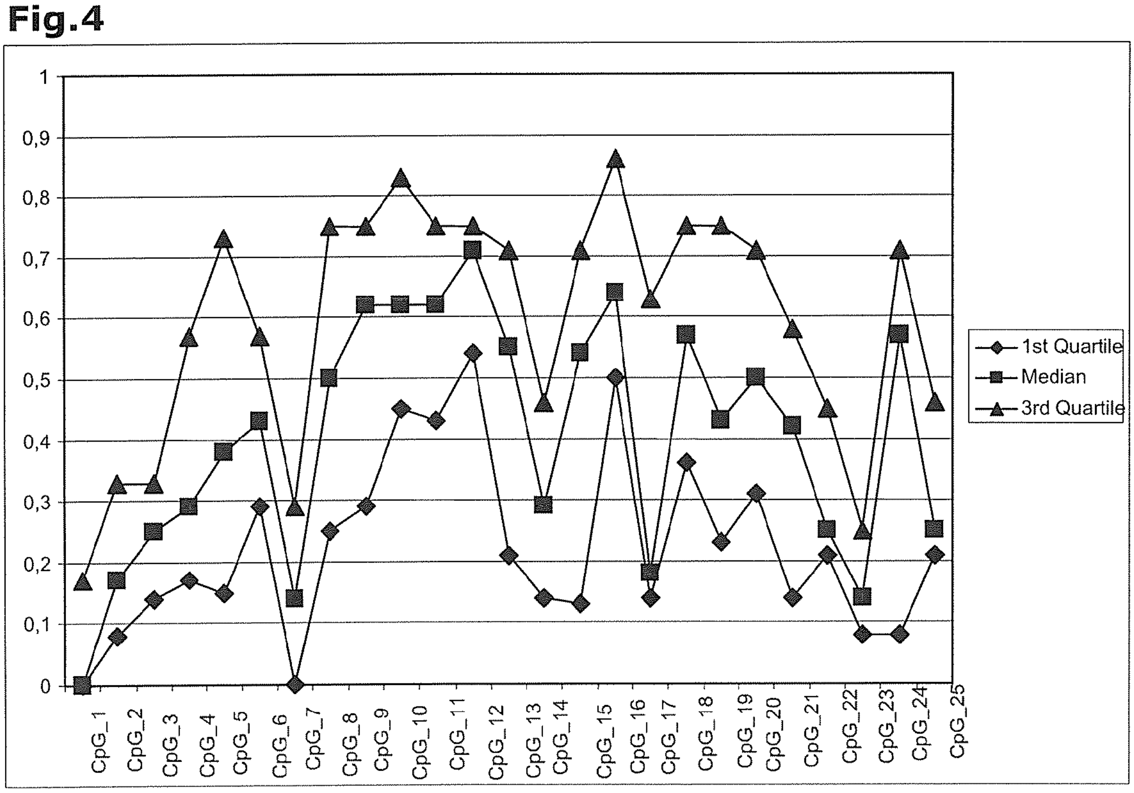
1
0,9
0,8
0,7
0,6
0,5
0,4
0,3
0,2
0,1
0
CpG_1
CpG_2
CpG_3
CpG_4
CpG_5
CpG_6
CpG_7
CpG_8
CpG_9
CpG_10
CpG_11
CpG_12
CpG_13
CpG_14
CpG_15
CpG_16
CpG_17
CpG_18
CpG_19
CpG_20
CpG_21
CpG_22
CpG_23
CpG_24
CpG_25
1st Quartile
Median
3rd Quartile

Fig.5

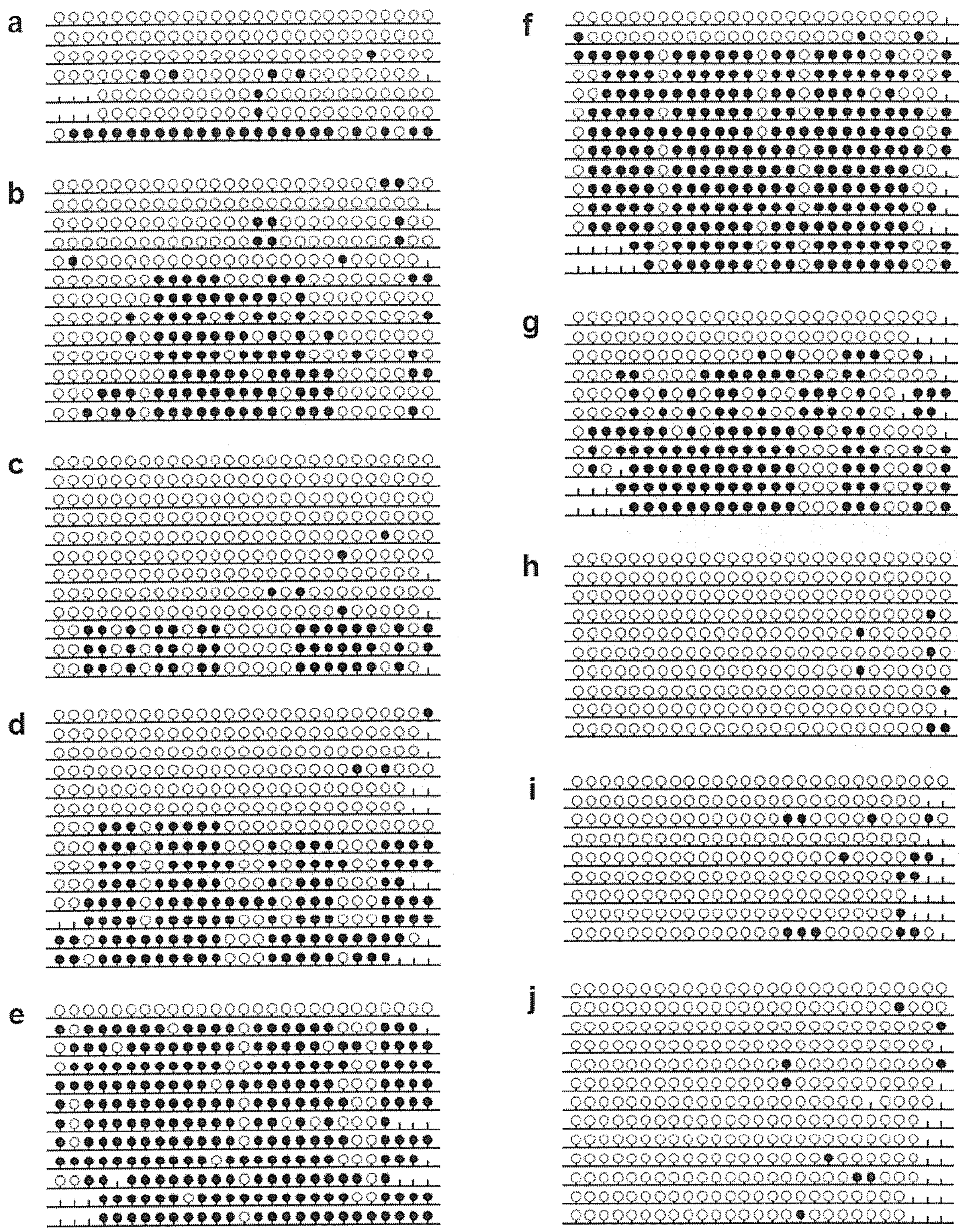


Fig.6

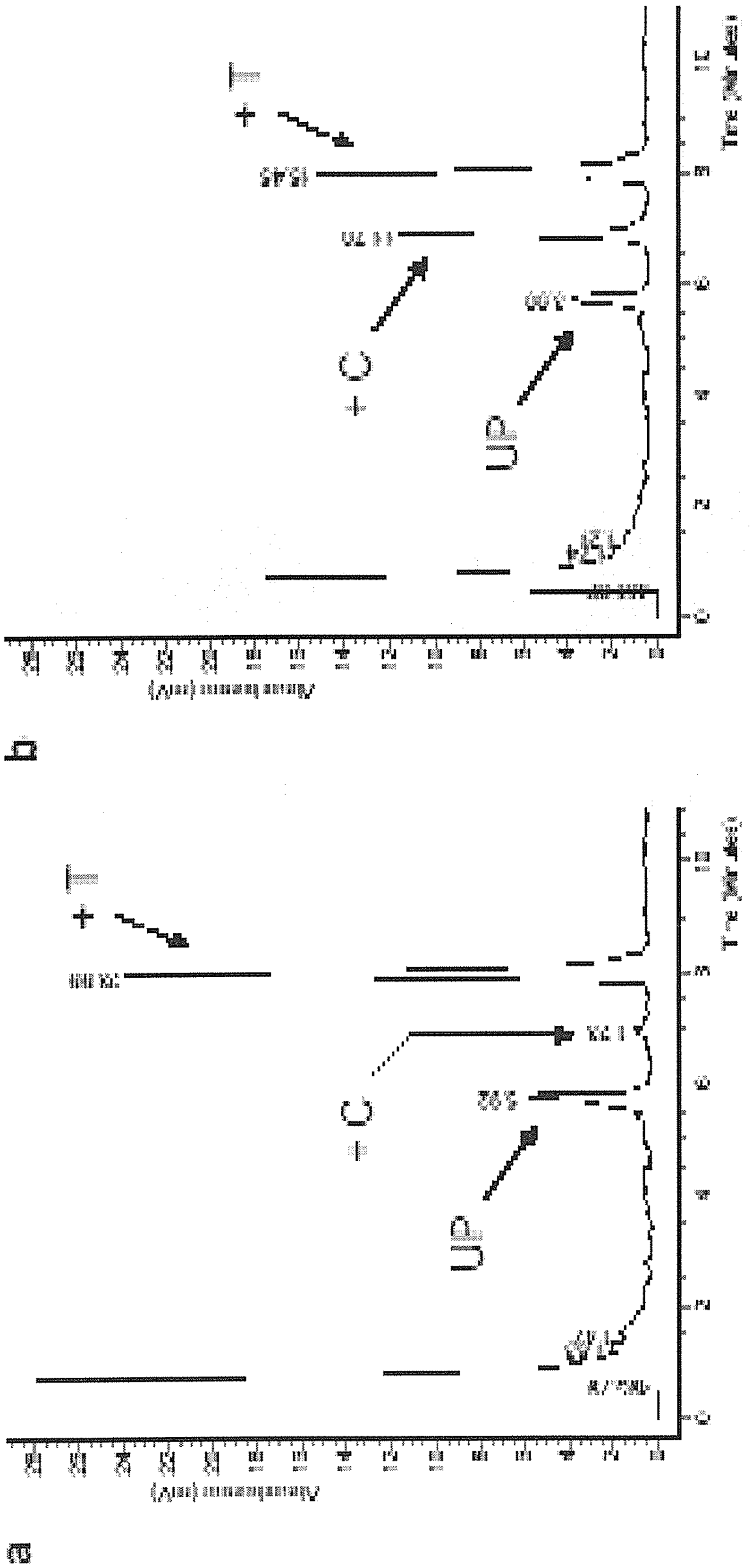


Fig.7

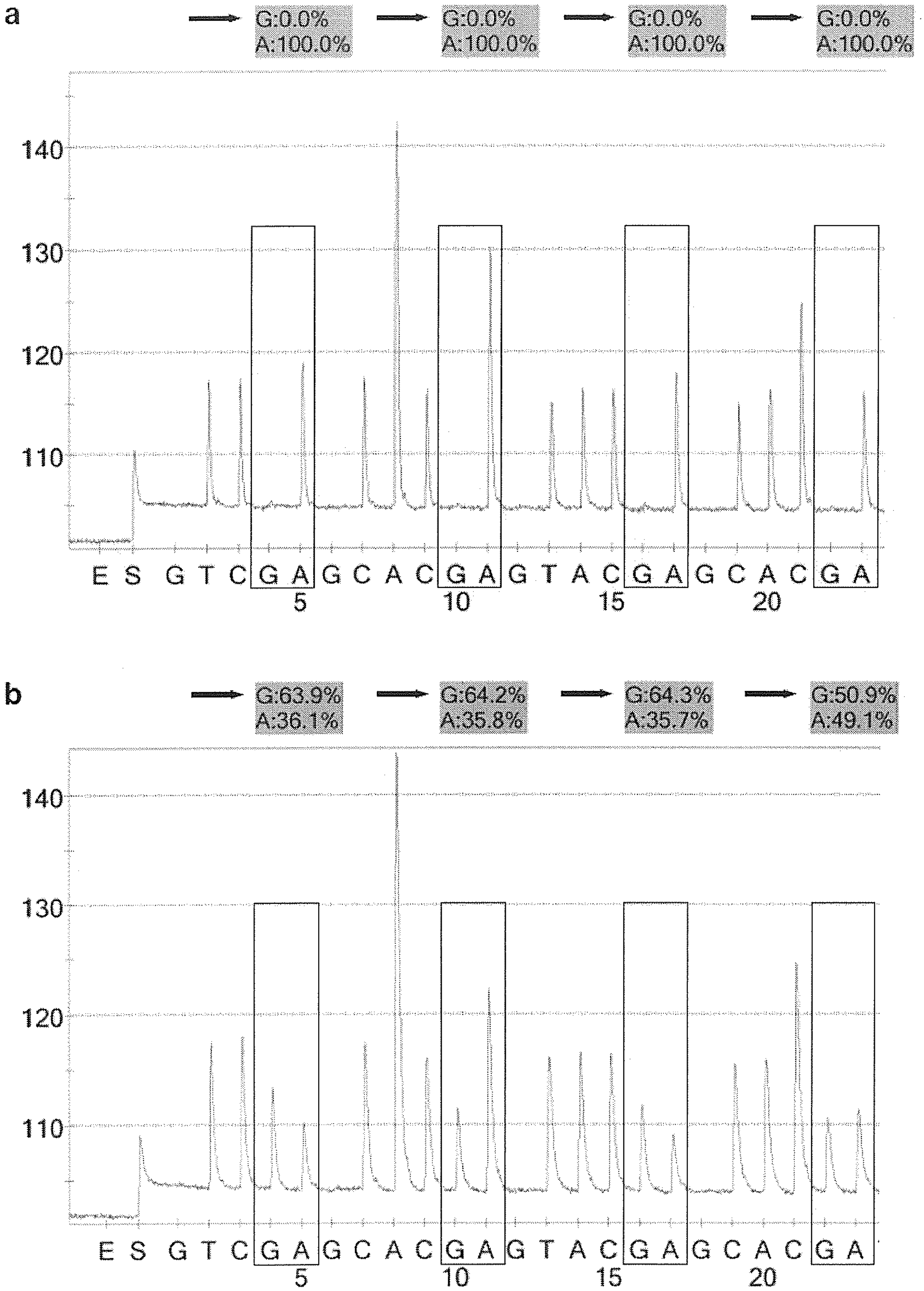
a
G:0.0%
A:100.0%
G:0.0%
A:100.0%
G:0.0%
A:100.0%
G:0.0%
A:100.0%
140
130
120
110
E S G T C G A G C A C G A G T A C G A G C A C G A
5
10
15
20
b
G:63.9%
A:36.1%
G:64.2%
A:35.8%
G:64.3%
A:35.7%
G:50.9%
A:49.1%
140
130
120
110
E S G T C G A G C A C G A G T A C G A G C A C G A
5
10
15
20

Fig.8

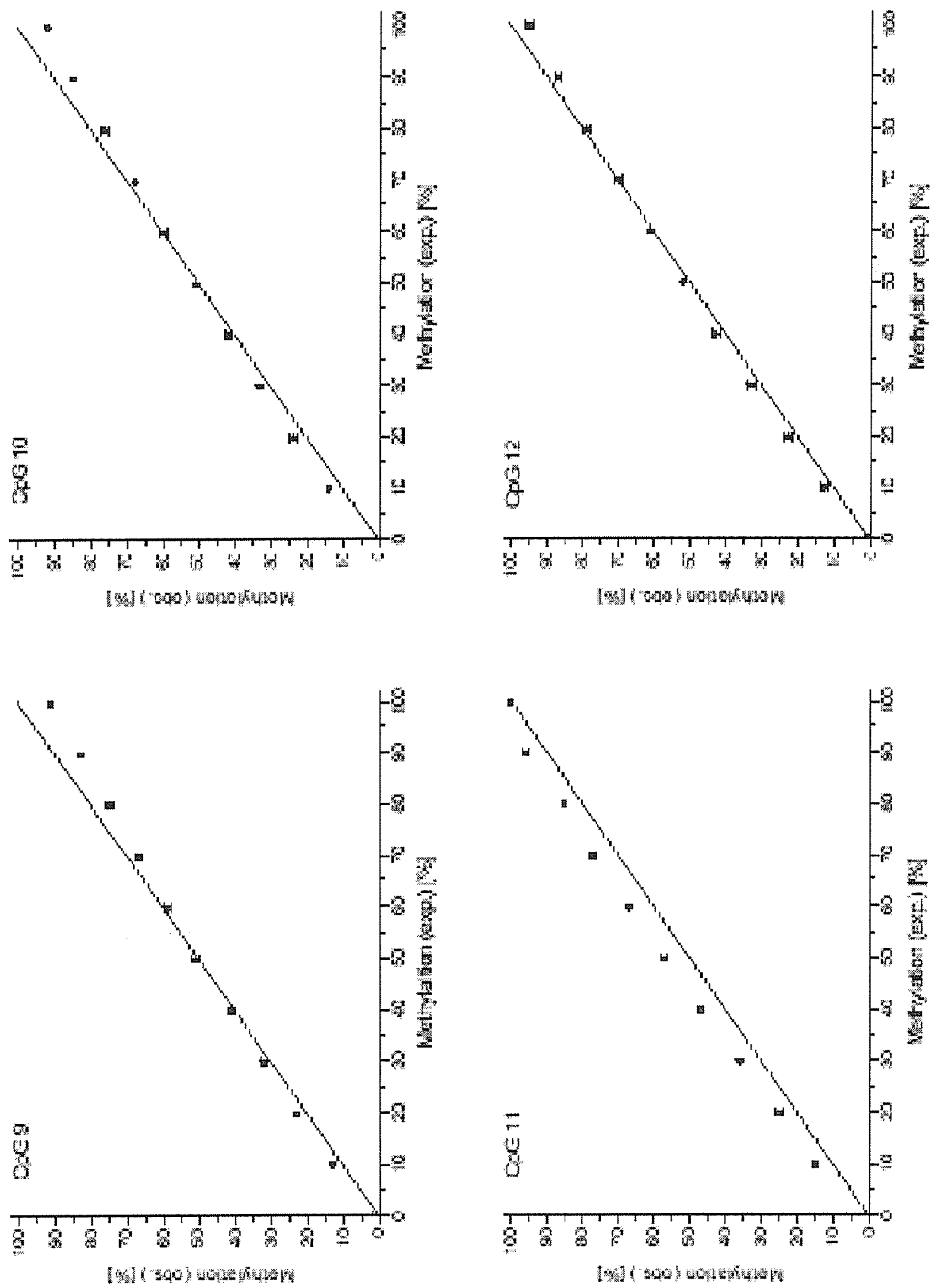


Fig.9

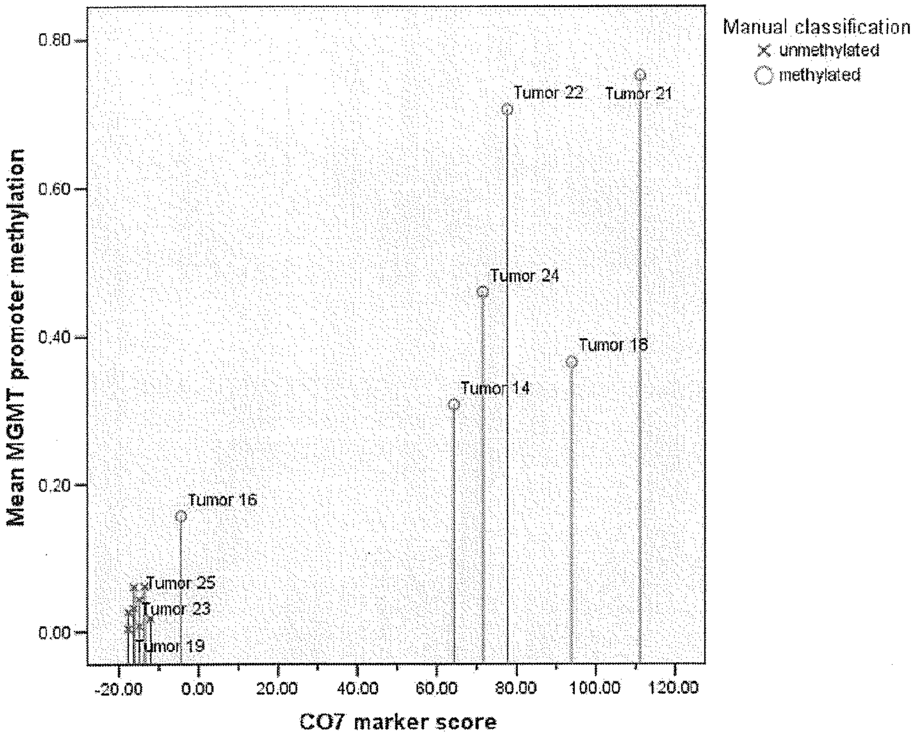


Fig.10a

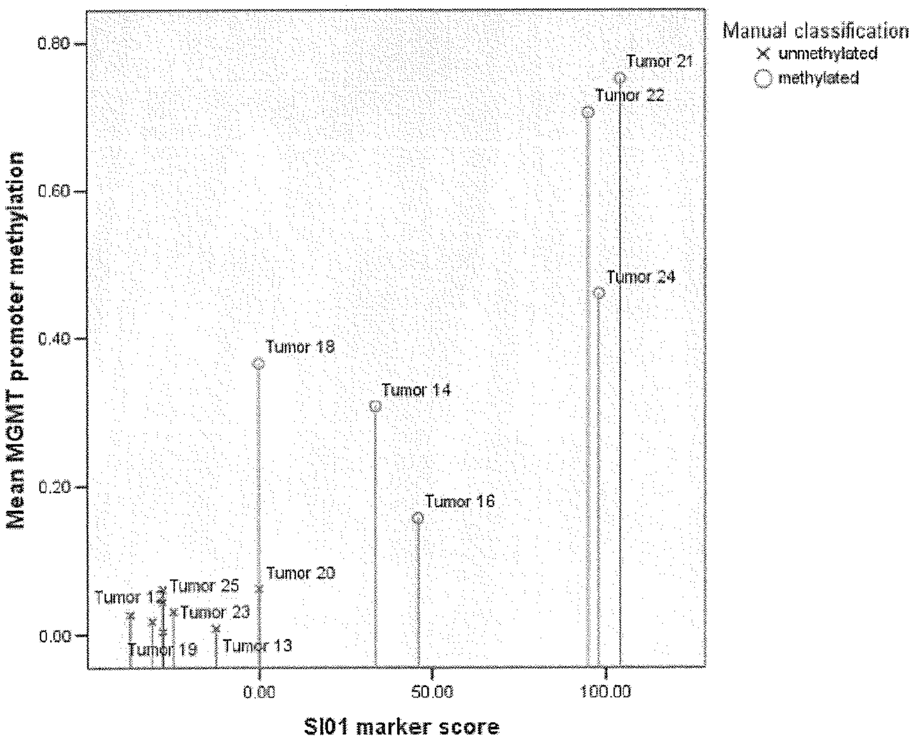


Fig.10b

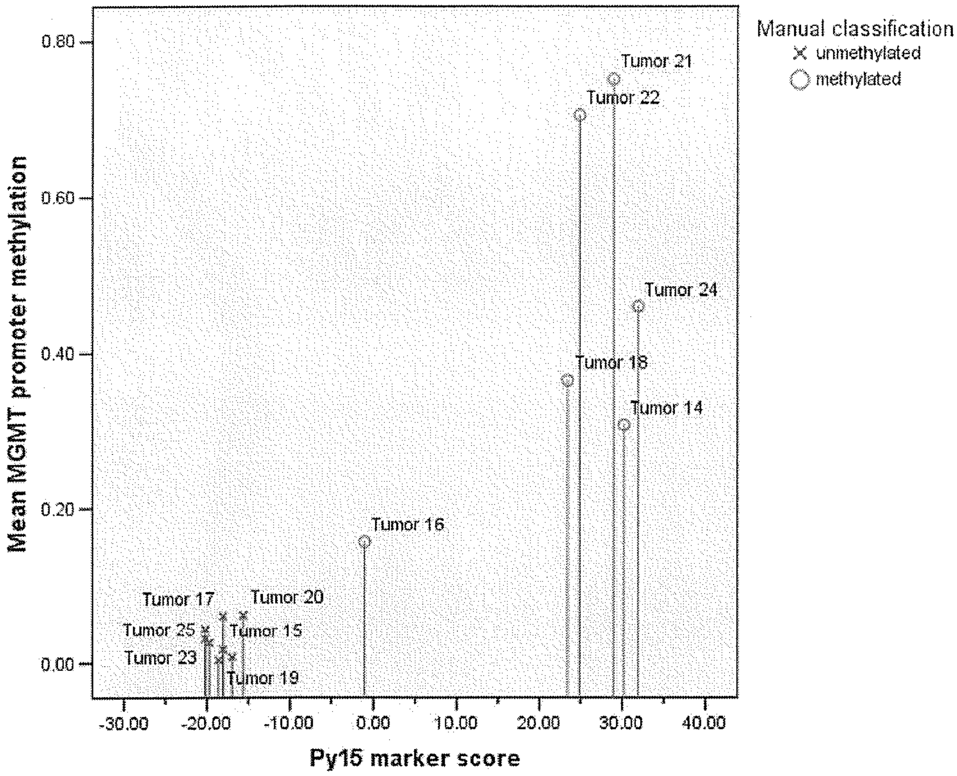
Manual classification
unmethylated
methylated
Tumor 21
Tumor 22
Tumor 24
Tumor 18
Tumor 14
Tumor 16
Tumor 17
Tumor 20
Tumor 25
Tumor 15
Tumor 23
Tumor 19
0.80
0.60
0.40
0.20
0.00
Mean MGMT promoter methylation
-30.00
-20.00
-10.00
0.00
10.00
20.00
30.00
40.00
Py15 marker score

**Fig.10c**

European Patent Office

# EUROPEAN SEARCH REPORT

Application Number
EP 06 12 0609

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YAMAMOTO T ET AL: "METHYLATION ASSAY BY NUCLEOTIDE INCORPORATION: A QUANTITATIVE ASSAY FOR REGIONAL CPG METHYLATION DENSITY" BIOTECHNIQUES, INFORMA LIFE SCIENCES PUBLISHING, WESTBOROUGH, MA, US, vol. 36, no. 5, 2004, pages 846-854, XP001247943 ISSN: 0736-6205 | 1-4,11, 15,16 | INV. C12Q1/68 |
| Y | * the whole document * | 5-10,12, 13 | |
| | ----- | | |
| X | WO 2004/086949 A (WAYNE JOHN CANCER INST [US]; HOON DAVE S B [US]; TABACK BRET [US]) 14 October 2004 (2004-10-14) * page 3, line 17 - page 4, line 26 * * page 9, line 7 - line 14 * * example 3 * * claims 23-31 * | 1-4,11, 14-16 | |
| Y | | 5-10,12, 13 | |
| | ----- | | TECHNICAL FIELDS SEARCHED (IPC) |
| | -/-- | | C12Q |

The present search report has been drawn up for all claims

| Place of search | Date of completion of the search | Examiner |
|---|---|---|
| Munich | 14 February 2007 | Ulbrecht, Matthias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

European Patent Office

# EUROPEAN SEARCH REPORT

Application Number
EP 06 12 0609

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MATSUKURA S ET AL: "CpG methylation of MGMT and hMLH1 promoter in hepatocellular carcinoma associated with hepatitis viral infection." BRITISH JOURNAL OF CANCER 24 FEB 2003, vol. 88, no. 4, 24 February 2003 (2003-02-24), pages 521-529, XP002419892 ISSN: 0007-0920 * abstract * * page 524, right-hand column, last paragraph - page 425, left-hand column, paragraph 2 * * page 525, right-hand column, last paragraph * * page 527, right-hand column, last paragraph * * figures 2,3 * * table 1 * | 1-4,11, 14,15 | |
| Y | | 5-10,12, 13 | TECHNICAL FIELDS SEARCHED (IPC) |
| | ----- | | |
| X | BAE S I ET AL: "Inactivation of O6-methylguanine-DNA methyltransferase by promoter CpG island hypermethylation in gastric cancers." BRITISH JOURNAL OF CANCER 17 JUN 2002, vol. 86, no. 12, 17 June 2002 (2002-06-17), pages 1888-1892, XP002419893 ISSN: 0007-0920 * abstract * * page 1889, left-hand column, last paragraph - right-hand column, paragraph 2 * * figure 3 * | 1-4,11, 14-16 | |
| Y | | 5-10,12, 13 | |
| | ----- -/-- | | |

The present search report has been drawn up for all claims

| Place of search | Date of completion of the search | Examiner |
|---|---|---|
| Munich | 14 February 2007 | Ulbrecht, Matthias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2

EPO FORM 1503 03.82 (P04C01)

European Patent Office

# EUROPEAN SEARCH REPORT

Application Number
EP 06 12 0609

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br><br><br>Y | EP 1 416 056 A (NISSHIN SPINNING [JP]) 6 May 2004 (2004-05-06)<br>* example 1 *<br>* figure 2 *<br>* sequences 1-13 *<br>----- | 1-4,11, 15,16<br><br><br><br>5-10, 12-14 | |
| X,D<br><br><br><br><br><br><br><br><br>Y | XIONG Z AND LAIRD P W: "COBRA: a sensitive and quantitative DNA methylation assay"<br>NUCLEIC ACIDS RESEARCH, OXFORD UNIVERSITY PRESS, SURREY, GB,<br>vol. 25, no. 12, 1997, pages 2532-2534, XP002106407<br>ISSN: 0305-1048<br>* the whole document *<br>----- | 16<br><br><br><br><br><br><br><br><br>5,8-10, 15 | |
| D,Y | WO 02/27019 A1 (UNIV JOHNS HOPKINS MED [US]; BAYLIN STEPHEN B [US]; HERMAN JAMES G [US) 4 April 2002 (2002-04-04)<br>* example 1 *<br>* claims 1-19,36,39 *<br>* sequences 1-4 *<br>----- | 5-10, 12-14 | TECHNICAL FIELDS SEARCHED (IPC) |
| Y,D | TOST J ET AL: "ANALYSIS AND QUANTIFICATION OF MULTIPLE METHYLATION VARIABLE POSITIONS IN CPG ISLANDS BY PYROSEQUENCING"<br>BIOTECHNIQUES, INFORMA LIFE SCIENCES PUBLISHING, WESTBOROUGH, MA, US,<br>vol. 35, no. 1, July 2003 (2003-07), pages 152-154,156, XP001156833<br>ISSN: 0736-6205<br>* the whole document *<br>-----<br>-/-- | 5-7,15 | |

The present search report has been drawn up for all claims

| Place of search | Date of completion of the search | Examiner |
|---|---|---|
| Munich | 14 February 2007 | Ulbrecht, Matthias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

European Patent Office

# EUROPEAN SEARCH REPORT

Application Number
EP 06 12 0609

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | PARK T J ET AL: "Methylation of O(6)-methylguanine-DNA methyltransferase gene is associated significantly with K-ras mutation, lymph node invasion, tumor staging, and disease free survival in patients with gastric carcinoma." CANCER 1 DEC 2001, vol. 92, no. 11, 1 December 2001 (2001-12-01), pages 2760-2768, XP002419894 ISSN: 0008-543X * abstract * * page 2762, left-hand column, last paragraph * * page 2764, left-hand column, last paragraph - right-hand column, paragraph 1 * * page 2765, left-hand column, last paragraph - page 2767, left-hand column, paragraph 1 * * figure 3 * ----- | 1-16 | |

TECHNICAL FIELDS SEARCHED (IPC)

The present search report has been drawn up for all claims

2

| Place of search | Date of completion of the search | Examiner |
|---|---|---|
| Munich | 14 February 2007 | Ulbrecht, Matthias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 06 12 0609

This annex lists the patent family members relating to the patent documents cited in the above-mentioned European search report.
The members are as contained in the European Patent Office EDP file on
The European Patent Office is in no way liable for these particulars which are merely given for the purpose of information.

14-02-2007

| Patent document cited in search report | Publication date | Patent family member(s) | Publication date |
|---|---|---|---|
| WO 2004086949 A | 14-10-2004 | NONE | |
| EP 1416056 A | 06-05-2004 | CA 2442438 A1 | 04-04-2004 |
| | | US 2005089870 A1 | 28-04-2005 |
| WO 0227019 A1 | 04-04-2002 | AU 9651101 A | 08-04-2002 |
| | | CA 2422890 A1 | 04-04-2002 |
| | | EP 1328656 A1 | 23-07-2003 |
| | | JP 2004509643 T | 02-04-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0227019 A


### Non-patent literature cited in the description


- **SOEJIMA H et al.** *Biochem. Cell Biol.,* 2005, vol. 83, 429-437
- **GARDINER-GARDEN, M ; FROMMER, M.** *J. Mol. Biol.,* 1987, vol. 196 (2), 261-282
- **PALMISANO, WA et al.** *Cancer Res.,* 2000, vol. 60, 5954-5958
- **SAMBROOK et al.** Molecular Cloning: A Laboratory Manual. Cold Spring Harbor Laboratory Press, 2001
- **AUSUBEL et al.** Current Protocols in Molecular Biology, Current Protocols in Immunology, Current Protocols in Protein Science, and Current Protocols in Cell Biology. Wiley Interscience, 1987
- **LUDLUM DB.** *Mutat. Res,* 1990, vol. 233, 117-26
- **PEGG AE.** *Mutat. Res,* 2000, vol. 462, 83-100
- **PEGG AE ; DOLAN ME ; MOSCHEL RC.** *Prog Nucleic Acid Res Mol Biol,* 1995, vol. 51, 167-223
- **WIBLEY JEA ; PEGG AE ; MOODY PCE.** *Nucl Acids Res,* 2000, vol. 28, 393-401
- **KARRAN P ; BIGNAMI M.** *Bioessays,* 1994, vol. 16, 833-39
- **MARGISON GP ; POVEY AC ; KAINA B ; SANTIBÁÑEZ KOREF MF.** *Carcinogenesis,* 2003, vol. 4, 625-35
- **CITRON M et al.** *Cancer Res,* 1991, vol. 51, 4131-34
- **SILBER JR ; MUELLER BA ; EWERS TG ; BERGER MS.** *Cancer Res,* 1993, vol. 53, 3416-20
- **ESTELLER M ; HAMILTON SR ; BURGER PC ; BAYLIN SB ; HERMAN, JG.** *Cancer Res,* 1999, vol. 59, 793-97
- **FRITZ G ; TANO K ; MITRA S ; KAINA B.** *Mol Cell Biol,* 1991, vol. 11, 4660-68
- **GROMBACHER T ; MITRA S ; KAINA B.** *Carninogenesis,* 1996, vol. 17, 2329-36
- **ESTELLER M et al.** *N Engl J Med,* 2000, vol. 343, 1350-54
- **PAZ MF et al.** *Clin Cancer Res,* 2004, vol. 10, 4933-38
- **GERSON SL.** *Nat Rev Cancer,* 2004, vol. 4, 296-307
- **HEGI ME et al.** *N Engl J Med,* 2005, vol. 352, 997-1003
- **HERMAN JG ; GRAFF JR ; MYÖHÄNEN S ; NELKIN BD ; BAYLIN SB.** *Proc. Natl Acad Sci USA,* 1986, vol. 93, 9821-26
- **DERKS S ; LENTJES, MHFM ; HELLEBREKERS, DMEI ; DE BRUÏNE, AP ; HERMAN, JG ; VAN ENGELAND, M.** *Cell Oncol,* 2004, vol. 26, 291-99
- **XIONG Z ; LAIRD P. W.** *Nucl Acids Res,* 1997, vol. 25, 2532-34
- **EL-MAARRI O ; HERBINIAUX U ; WALTER J ; OLDENBURG J.** *Nucl Acids Res,* 2002, vol. 30, e25
- **TOST J ; DUNKER J ; GUT IG.** *Biotechniques,* 2003, vol. 35, 152-56
- **KLEIHUES P ; WEBSTER KC.** Pathology and Genetics of Tumours of the Nervous System. World Health Organization Classification of Tumours. Oxford University Press, 2000
- **SAMBROOK J ; FRITSCH EF ; MANIATIS T.** Molecular cloning. Cold Spring Harbor Laboratory Press, 1989
- **PALMISANO WA et al.** *Cancer Res,* 2000, vol. 60, 5954-58
- **BOCK C ; REITHER S ; MIKESKA T ; PAULSEN M ; WALTER J ; LENGAUER T.** *Bioinformatics,* 2005, vol. 21, 4067-68
- **WITTEN IH ; FRANK E.** *Data mining: practical machine learning tools and techniques with Java implementations,* 2000, 371
- **LARSEN F ; GUNDERSEN G ; LOPEZ R ; PRYDZ H.** *Genomics,* 1992, vol. 13, 1095-107
- **SHAW RJ ; AKUFO-TETTEH EK ; RISK JM ; FIELD JK ; LILOGLOU T.** *Nucl Acids Res,* 2006, vol. 34, e78